# EUROPEAN PATENT APPLICATION

(11) **EP 1 362 850 A1**
(43) Date of publication of application: **19.11.2003**
(21) Application number: 02700611.3
(22) Date of filing: 20.02.2002
(51) Int. Cl.: C07D 213/64, C07D 213/69, C07D 401/12, C07D 409/12, C07D 405/12, C07D 413/12, C07D 417/12, C07D 213/70, C07D 213/74, C07D 213/73, C07D 213/84, C07D 213/53, A01N 43/40, A01N 43/54, A01N 43/60, A01N 43/58

(54) **OXIME ETHER COMPOUND AND AGRICULTURAL OR HORTICULTURAL BACTERICIDE**

(30) Priority: 20.02.2001 JP 2001043500
(71) Applicant: NIPPON SODA CO., LTD., Chiyoda-ku, Tokyo 100-8165 (JP)
(72) Inventor: NAKAGAWA, Yuki, Gainesville, FL 32606 (US); MITANI, Akira, c/o Nippon Soda Co. Ltd., Odawara-shi, Kanagawa 250-0280 (JP); SANO, Hiroshi, c/o Nippon Soda Co. Ltd., Odawara-shi, Kanagawa 250-0280 (JP); HAMAMURA, Hiroshi, c/o Nippon Soda Co. Ltd., Odawara-shi, Kanagawa 250-0280 (JP); ANDO, Takahiro, c/o Nippon Soda Co. Ltd., Odawara-shi, Kanagawa 250-0280 (JP); SUGIURA, Tadashi, c/o Nippon Soda Co. Ltd., Odawara-shi, Kanagawa 250-0280 (JP); ITO, Shuichi, c/o Nippon Soda Co. Ltd., Odawara-shi, Kanagawa 250-0280 (JP)
(74) Representative: Grünecker, Kinkeldey, Stockmair & Schwanhäusser Anwaltssozietät
(86) International application number: JP0201450
(87) International publication number: WO02066434

(57) **Abstract**

An oxime ether compound represented by the formula [I] or a salt thereof which have excellent bactericidal activity and are useful as an agricultural or horticultural fungicide. [I] (In the formula, R¹ represents halogeno, hydroxy, C₁₋₆ alkyl, C₁₋₆ haloalkyl, C₁₋₆ alkoxy, C₁₋₆ alkyl substituted by C₁₋₆ alkoxy, cyano, nitro, C₂₋₆ alkenyl, C₂₋₆ haloalkenyl, C₂₋₆ alkynyl, amino, mono(C₁₋₆ alkyl)amino, di(C₁₋₆ alkyl)amino, C₃₋₆ cycloalkyl, carboxyl, C₁₋₆ alkoxycarbonyl, etc.; R² represents hydrogen, C₁₋₆ alkyl, C₃₋₆ cycloalkyl, C₁₋₆ haloalkyl, etc.; R³ and R⁴ each represents hydrogen or C₁₋₆ alkyl; k is an integer of 1 to 4; and A represents the heterocyclic group shown in the description.)

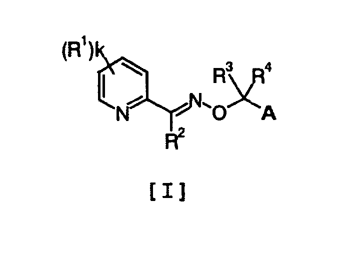

## Description

### FIELD OF INVENTION:

The present invention relates to novel oxime ether compounds and to agricultural or horticultural fungicides containing the said compounds as active ingredients.

### BACKGROUND ART:

A large number of chemicals have been used to control insect pests and diseases on crops when agricultural or horticultural crops are cultivated. Many of such chemicals are, however, not always satisfactory controlling agents because of insufficient efficacy, restrictions on their use due to the appearance of chemical-resistant strains of the diseases and insect pests, chemical injuries or pollution of plants, or strong toxicity to humans, domestic animals and fish. Therefore, there are strong needs for chemicals that have less of the drawbacks mentioned above and that can be used safely.

As for compounds relating to those of the present invention, certain types of oxime ether compounds are disclosed to have insecticidal and acaricidal activities in EP 4754, EP 24888, WO 93/21157 and others.

Japanese Patent Laid-open No. Hei 9-3047 describes that oxime ether compounds including the compound represented by the following formula are useful as fungicides. However, compounds with non-substituted 3-thienyl group are only described as compounds of Formula [I] shown later where A is a heterocyclic group.

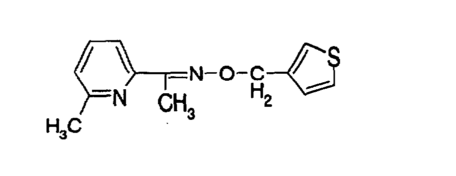

### DISCLOSURE OF THE INVENTION:

It is an object of the present invention to provide novel oxime ether compounds that can be synthesized advantageously at industrial scales and become excellent, effective agricultural or horticultural fungicides.

The present invention relates to an oxime ether compound represented by Formula [I] 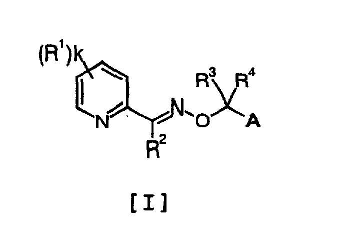 [wherein, R¹ is halogen, hydroxyl, C₁₋₆ alkyl, C₁₋₆ haloalkyl, C₁₋₆ alkoxy, C₁₋₆ alkoxy C₁₋₆ alkyl, cyano, nitro, C₂₋₆ alkenyl, C₂₋₆ haloalkenyl, C₂₋₆ alkynyl, amino, mono- or di-C₁₋₆ alkylamino, C₃₋₆ cycloalkyl, carboxyl, C₁₋₆ alkoxycarbonyl, C₁₋₆ alkylthio, C₁₋₆ alkylsulfinyl or C₁₋₆ alkylsulfonyl; R² is hydrogen, C₁₋₆ alkyl, C₃₋₆ cycloalkyl, C₁₋₆ haloalkyl, amino or cyano; R³ and R⁴ are, the same or different, hydrogen or C₁₋₆ alkyl; k is an integer of 1 to 4; when k is 2 or larger, R¹ may be the same or different; A is a heterocyclic group represented by the following Formulae A-1 to A-12 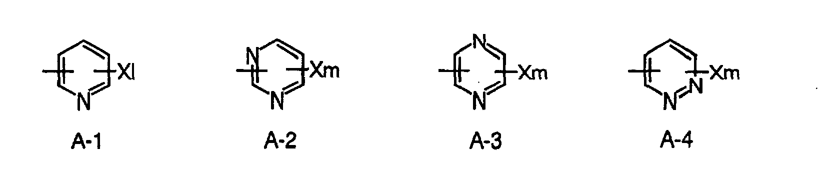 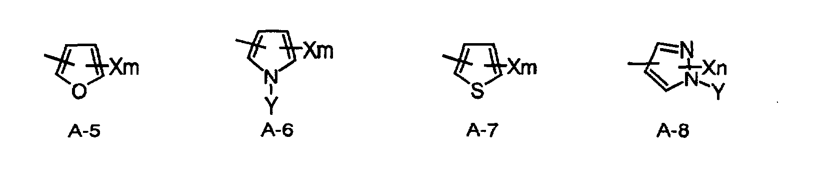 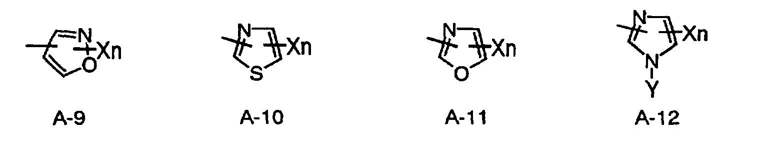 (wherein, X is halogen, C₁₋₆ alkyl, C₁₋₆ haloalkyl, hydroxyl, C₁₋₆ alkoxy, cyano, amino, mono-C₁₋₆ alkylamino, di-C₁₋₆ alkylamino, C₃₋₆ cycloalkyl, C₁₋₆ alkylcarbonyloxy or C₁₋₆ alkylthio; 1 is 0 or an integer of 1 to 4; m is 0 or an integer of 1 to 3; n is 0, 1 or 2; when 1, m and n are 2 or more, X may be the same or different; but when A is A-7, m is not 0; and Y is hydrogen or C₁₋₆ alkyl)] or a salt thereof. It also relates to an agricultural or horticultural fungicide containing one or more of the said compounds as active ingredients.

The present invention is described in detail.

In Formula [I], R¹ is halogen such as fluorine, chlorine, bromine or iodine; C₁₋₆ alkyl such as methyl, ethyl, n-propyl, isopropyl, n-butyl, sec-butyl, isobutyl, t-butyl, n-pentyl or n-hexyl; C₁₋₆ haloalkyl such as chloromethyl, fluoromethyl, bromomethyl, dichloromethyl, difluoromethyl, dibromomethyl, trichloromethyl, trifluoromethyl, tribromomethyl, trichloroethyl, trifluoroethyl or pentafluoroethyl; C₁₋₆ alkoxy such as methoxy, ethoxy, n-propoxy, isopropoxy, n-butoxy, sec-butoxy, isobutoxy or t-butoxy; C₁₋₆ alkoxy C₁₋₆ alkyl such as methoxymethyl, ethoxymethyl, 1-methoxyethyl or 2-methoxyethyl; C₂₋₆ alkenyl such as ethenyl, 1-propenyl, 2-propenyl, 1-butenyl, 2-butenyl, 3-butenyl, 1-methyl-2-propenyl, 2-methyl-2-propenyl, 1-pentenyl, 2-pentenyl, 3-pentenyl, 4-pentenyl, 1-methyl-2-butenyl, 2-methyl-2-butenyl, 1-hexenyl, 2-hexenyl, 3-hexenyl, 4-hexenyl or 5-hexenyl; C₂₋₆ haloalkenyl such as 3-chloro-2-propenyl, 4-chloro-2-butenyl, 4,4-dichloro-3-butenyl, 4,4-difluoro-3-butenyl or 3,3-dichloro-2-propenyl; C₂₋₆ alkynyl such as ethynyl, 1-propynyl, 2-propynyl, 1-butynyl, 2-butynyl, 3-butynyl, 1-methyl-2-propynyl, 2-methyl-3-butynyl, 1-pentynyl, 2-pentynyl, 3-pentynyl, 4-pentynyl, 1-methyl-2-butynyl, 2-methyl-3-pentynyl, 1-hexynyl or 1,1-dimethyl-2-butynyl; mono- or di-C₁₋₆ alkylamino such as methylamino, ethylamino, n-propylamino, isopropylamino, n-butylamino, isobutylamino, sec-butylamino, t-butylamino, 1-methylbutylamino, n-pentylamino, dimethylamino, diethylamino, di-n-propylamino, di-n-butylamino, ethylisopropylamino or methyl-n-propylamino; C₃₋₆ cycloalkyl such as cyclopropyl, 1-methylcyclopropyl, 2,2,3,3-tetramethylcyclopropyl, cyclobutyl, cyclopentyl, 1-methylcyclopentyl, cyclohexyl, 1-methylcyclohexyl or 4-methylcyclohexyl; C₁₋₆ alkoxycarbonyl such as methoxycarbonyl, ethoxycarbonyl, n-propoxycarbonyl, isopropoxycarbonyl, n-butoxycarbonyl or t-butoxycarbonyl; C₁₋₆ alkylthio such as methylthio, ethylthio, n-propylthio, isopropylthio, n-butylthio, isobutylthio, sec-butylthio or t-butylthio; C₁₋₆ alkylsulfinyl such as methylsulfinyl, ethylsulfinyl, n-propylsulfinyl or n-butylsulfinyl; or C₁₋₆ alkylsulfonyl such as methylsulfonyl, ethylsulfonyl, n-propylsulfonyl or n-butylsulfonyl.

R² is C₁₋₆ alkyl such as methyl, ethyl, n-propyl, isopropyl, n-butyl, sec-butyl, isobutyl, t-butyl, n-pentyl or n-hexyl; C₃₋₆ cycloalkyl such as cyclopropyl, 1-methylcyclopropyl, 2,2,3,3-tetramethylcyclopropyl, cyclobutyl, cyclopentyl, 1-methylcyclopentyl, cyclohexyl, 1-methylcyclohexyl or 4-methylcyclohexyl; C₁₋₆ alkoxycarbonyl such as methoxycarbonyl, ethoxycarbonyl, n-propoxycarbonyl, isopropoxycarbonyl, n-butoxycarbonyl or t-butoxycarbonyl; or C₁₋₆ haloalkyl such as chloromethyl, fluoromethyl, bromomethyl, dichloromethyl, difluoromethyl, dibromomethyl, trichloromethyl, trifluoromethyl, tribromomethyl, trichloroethyl, trifluoroethyl or pentafluoroethyl.

R³ and R⁴ are, independently, C₁₋₆ alkyl such as methyl, ethyl, n-propyl, isopropyl, n-butyl, sec-butyl, isobutyl, t-butyl, n-pentyl or n-hexyl.

Examples of heterocyclic groups selected from Formulae A-1 to A-12 include 2-pyridyl, 3-pyridyl, 4-pyridyl, 4-pyrimidyl, 5-pyrimidyl, 2-pyrazinyl, 3-pyridazinyl, 4-pyridazinyl, 1-furyl, 2-furyl, 2-pyrrolyl, 3-pyrrolyl, 1-thienyl, 2-thienyl, 1-methyl-3-pyrrolyl, 3-pyrazolyl, 4-pyrazolyl, 5-pyrazolyl, 1-methyl-3-pyrazolyl, 1-methyl-4-pyrazolyl, 1-methyl-5-pyrazolyl, 3-isoxazolyl, 4-isoxazolyl, 5-isoxazolyl, 4-thiazolyl, 5-thiazolyl, 4-oxazolyl, 5-oxazolyl, 4-imidazolyl, 1-methyl-2-imidazolyl and 1-methyl-4-imidazolyl.

Substituents X and/or Y of these heterocyclic groups are optionally substituted.

X is halogen such as fluorine, chlorine, bromine or iodine; C₁₋₆ alkyl such as methyl, ethyl, n-propyl, isopropyl, n-butyl, sec-butyl, isobutyl or t-butyl; or C₁₋₆ haloalkyl such as chloromethyl, fluoromethyl, bromomethyl, dichloromethyl, difluoromethyl, dibromomethyl, trichloromethyl, trifluoromethyl, tribromomethyl, trichloroethyl, trifluoroethyl or pentafluoroethyl. Y is C₁₋₆ alkyl such as methyl, ethyl, n-propyl, isopropyl, n-butyl, sec-butyl, isobutyl or t-butyl.

The compounds of Formula [I] and their salts of the present invention have excellent fungicidal activities against a wide variety of mycetes, for example, fungi belonging to Oomycetes, Ascomycetes, Deuteromycetes and Basidiomycetes.

Compositions containing the compounds of the present invention as active ingredients can be used for controlling various plant diseases infesting agricultural and horticultural crops including flowers, ornamental plants, lawns and forage crops when they are cultivated by means of seed treatment, spraying on foliage, soil application, water surface application and other means.

The compounds are effective to control, for example, the following diseases:

| | |
|---|---|
| Sugar beet | Cercospora leaf spot *(Cercospora beticola)* |
| Ground nut | Leaf spot *(Mycosphaerella arachidis)* |
| | Late leaf spot *(Mycosphaerella berkeleyi)* |
| Cucumber | Powdery mildew *(Sphaerotheca fuliginea)* |
| | Gummy stem blight *(Mycosphaerella* melonis) |
| | Sclerotinia rot *(Sclerotinia sclerotiorum)* |
| | Gray mold *(Botrytis cinerea)* Scab *(Cladosporium cucumerinum)* |
| Tomato | Gray mold *(Botrytis cinerea)* |
| | Leaf mold *(Cladosporium fulvum)* |
| Eggplant | Gray mold *(Botrytis cinerea)* |
| | Black rot *(Corynespora melongenae)* |
| | Powdery mildew *(Erysiphe cichoracearum)* |
| Strawberry | Gray mold *(Botrytis cinerea)* |
| | Powdery mildew *(Sohaerotheca aphanis)* |
| Onion | Gray mold neck rot *(Botrytis allii)* |
| | Gray mold *(Botrytis cinerea)* |
| Kidney bean | Sclerotinia rot *(Sclerotinia* sclerotiorum) |
| | Gray mold (*Botrytis cinerea)* |
| Apple | Powdery mildew *(Podosphaera leucotricha)* |
| | Scab *(Venturia inaequalis)* |
| | Blossam blight (*Monilinia mali)* |
| Oriental persimmon | Powdery mildew *(Phyllactinia kakicola)* |
| | Anthracnose *(Gloeosporium kaki)* |
| | Angular leaf spot (*Cercospora kaki*) |
| Peach and cherry | Brown rot *(Monilinia fructicola)* |
| Grape | Gray mold *(Botrytis cinerea)* |
| | Powdery mildew *(Uncinula necator)* |
| | Ripe rot *(Glomerella cingulata)* |
| Pear | Scab *(Venturia nashicola)* |
| | Rust *(Gymnosporangium asiaticum)* |
| | Black spot *(Alternaria kikuchiana)* |
| Tea plant | Gray blight *(Pestalotia theae*) |
| | Anthracnose *(Colletotrichum theae- sinensis)* |
| Citrus | Scab *(Elsinoe fawcetti)* |
| | Blue mold *(Penicillium italicum*) |
| | Common green mold *(Penicillium digitatum)* |
| | Gray mold *(Botrytis cinerea)* |
| Barley | Powdery mildew *(Erysiphe graminis f.sp. hordei)* |
| | Loose smut *(Ustilago nuda)* |
| | Wheat's Scab *(Gibberella zeae)* |
| | Leaf rust *(Puccinia recondita)* |
| | Spot blotch *(Cochliobolus sativus)* |
| | Eye spot *(Pseudocercosporella herpotrichoides)* |
| | Glume blotch *(Leptosphaeria nodorum)* |
| | Powdery mildew *(Erysiphe graminis f.sp. tritici)* |
| | Snow mold *(Micronectriella nivalis)* |
| Paddy rice | Blast (*Pyricularia oryzae)* |
| | Sheath blight (*Rhizoctonia solani)* |
| | Bakanae disease (*Gibberella fujikuroi)* |
| | Helminthosporium leaf spot |
| | *(Cochliobolus niyabeanus)* |
| Tobacco | Sclerotinia rot *(Sclerotinia sclerotiorum)* |
| | Powdery mildew *(Erysiphe* |
| | *cichoracearum)* |
| Tulip | Gray mold (Botrytis cinerea) |
| Bent grass | Sclerotinia snow blight |
| | *(Sclerotinia borealis)* |
| Orchard grass | Powdery mildew *(Erysiphe graminis)* |
| Soybean | Purple speck *(Cercospora kikuchii)* |
| Potato and tomato | Downy mildew *(Phytophthora infestans)* |
| Cucumber | Downy mildew *(Pseudoperonospora cubensis)* |
| Grape | Downy mildew (*Plasmopara viticola)* |

In recent years, various pathogenic fungi have developed resistance to benzimidazole fungicides, dicarboxyimide fungicides and others. This has resulted in lack of efficacy of these chemicals. There are needs for fungicides effective to resistant strains. The compounds of the present invention have excellent fungicidal activities against sensitive strains as well as resistant ones.

The compounds of the present invention are effective against resistant strains (for example, resistant to thiophanate methyl, benomyl and carbendazim) of gray mold *(Botrytis cinerea),* sugar beet cercospora leaf spot (*Cercospora beticola*), apple scab (*Venturia inaequalis*) and pear scab (*Venturia nashicola),* and also against their sensitive strains.

Furthermore, the compounds of the present invention are effective on gray mold *(Botrytis cinerea)* strains resistant to dicarboxyimide fungicides (for example, vinclozolin, procymidone and iprodione) and also on sensitive strains.

Compositions containing the compounds of the present invention as active ingredients (fungicides for agricultural or horticultural use) are more preferably applied to diseases such as cercospora leaf spot of sugar beet, wheat powdery mildew, paddy rice blast, apple scab, kidney-bean gray mold and groundnut leaf spot.

In addition, the compounds of the present invention can be used as antifouling agents to prevent aquatic creatures from fouling structures in water such as the bottoms of ships and fishing nets.

### Process 1:

The compounds of the present invention can be produced according to the following method: 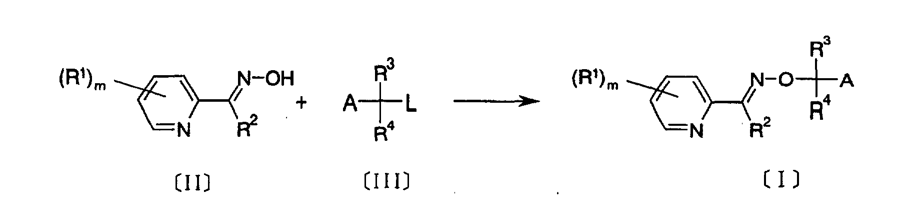 (wherein, R¹, R², R³, R⁴ and A are as defined above, and L is a leaving group including halogen such as chlorine, bromine or iodine; methanesulfonyloxy; or paratoluenesulfonyloxy).

A compound of Formula [I] is produced by stirring a compound of Formula [II] with a compound of Formula [III], without a solvent or preferably in a solvent, in the presence of a deacidifying agent such as a base at a reaction temperature of 0 to 150°C for 10 minutes to 24 hours.

Examples of solvents suitable to use for the reaction include ketones such as acetone and 2-butanone; ethers such as diethyl ether and tetrahydrofuran (THF); aromatic hydrocarbons such as benzene and toluene; alcohols such as methanol and ethanol; nitriles such as acetonitrile; amides such as N,N-dimethylformamide (DMF); dimethyl sulfoxide and water. Two or more of these solvents can be combined to be used as a mixed solvent.

Examples of suitable bases for use include inorganic bases such as sodium hydroxide, potassium hydroxide, potassium carbonate, sodium carbonate and sodium hydride; alkali metal alcolates such as sodium methylate and sodium ethylate; and organic bases such as pyridine, triethylamine and 1,8-diazabicyclo[5.4.0]undec-7-ene (DBU).

Among the compounds of Formula [II], which are starting materials for producing the compounds of the present invention, a compound of Formula [II'] where R² is hydrogen can be synthesized according to the following method: 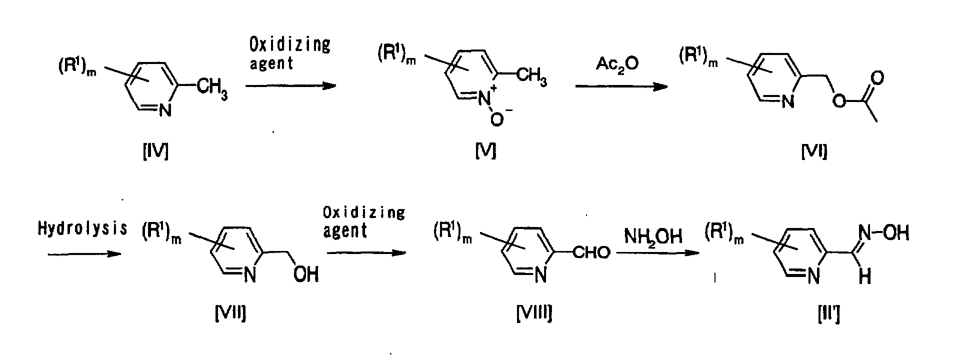 (wherein, R¹ and m are as defined above.)

A 2-methylpyridine represented by Formula [IV] is treated with an oxidizing agent, such as hydrogen peroxide or m-chloroperbenzoic acid, to derive to a pyridine-N-oxide of Formula [V], which then reacted with acetic anhydride to give a 2-acetoxymethylpyridine of Formula [VI].

Then, the 2-acetoxymethylpyridine of Formula [VI] is hydrolyzed to derive to a 2-hydroxymethylpyridine of Formula [VII], which is oxidized with an oxidizing agent, such as manganese dioxide, to synthesize a 2-pyridinecarboxyaldehyde represented by Formula [VIII]. Further, the 2-pyridinecarboxyaldehyde of Formula [VIII] is reacted with hydroxylamine to give a 2-pyridinecarboxyaldoxime of Formula [II'].

The following method may be applied to produce 2-pyridinecarboxyaldehydes of Formula [VIII]: 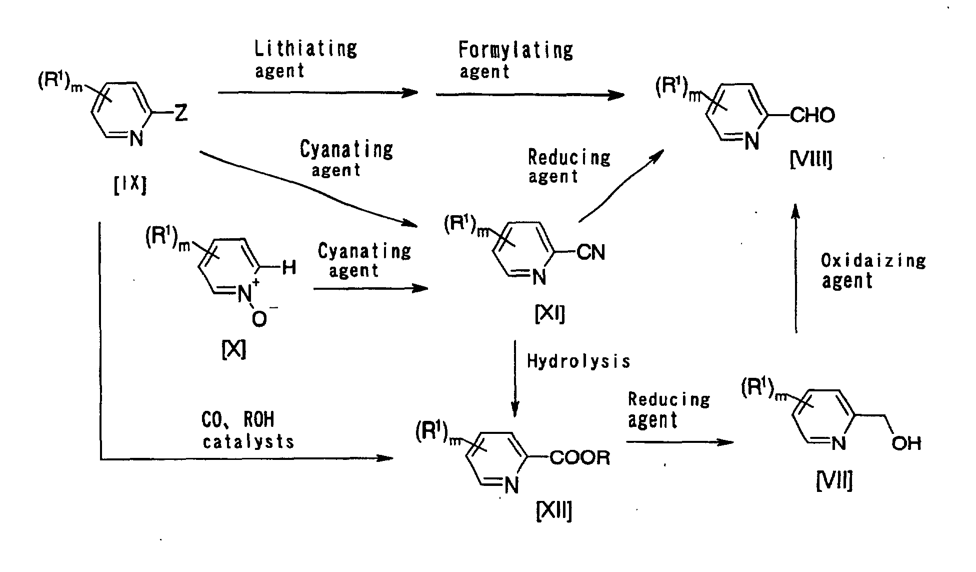 (wherein, R¹ and m are as defined above; Z is halogen such as chlorine, bromine or iodine; and R is lower alkyl.)

A 2-pyridinecarboxyaldehyde of Formula [VIII] can be produced by that a pyridine substituted with a halogen at Position 2 and represented by Formula [IX] is reacted with a lithiating agent, such as n-butyl lithium, for the lithiation at Position 2, followed by formylation at Position 2 with a formylating agent, such as DMF.

The 2-pyridinecarboxyaldehydes can also be produced by other processes: a pyridine substituted with a halogen at Position 2 and represented by Formula [IX] is treated with a cyanating agent, such as copper cyanide, or a pyridine N-oxide represented by Formula [X] that is not substituted at Position 2 or 6 is treated with a cyanating agent, such as trimethylsilyl cyanide (TMSCN), to derive to a cyanopyridine of Formula [XI], which is then reacted with a reducing agent, such as diisobutylaluminum hydride (DIBAL), to change the group at Position 2 to a formyl group; or the cyano group of a cyanopyridine of Formula [XI] is hydrolyzed to derive to a 2-pyridinecarboxylate of Formula [XII], which is then changed to a 2-hydroxymethylpyridine of Formula [XII] with a reducing agent, such as LiAlH₄ (LAH), followed by converting the 2-hydroxymethyl group with an oxidizing agent, such as manganese dioxide, to a formyl group.

Another method to produce 2-pyridinecarboxyaldehydes of Formula [VIII] is that a pyridine substituted with a halogen at Position 2 and represented by Formula [IX] is reacted with carbon monoxide in an alcohol in the presence of a catalyst, such as palladium complex, to derive a 2-pyridinecarboxylate of Formula [XII], which is then treated with a reducing agent, such as LiAlH₄, to convert to a 2-hydroxymethylpyridine of Formula [XII], followed by treating with an oxidizing agent, such as manganese dioxide, to change to a formyl group.

Among the compounds of Formula [II], which are starting materials for producing the compounds of the present invention, a compound where R² is lower alkyl can be synthesized according to a method disclosed in, for example, Japanese Patent Laid-open No. Hei 9-3047.

Among the compounds of Formula [II], which are starting materials for producing the compounds of the present invention, a compound represented by Formula [II''] where R² is amino can be synthesized according to the following method: 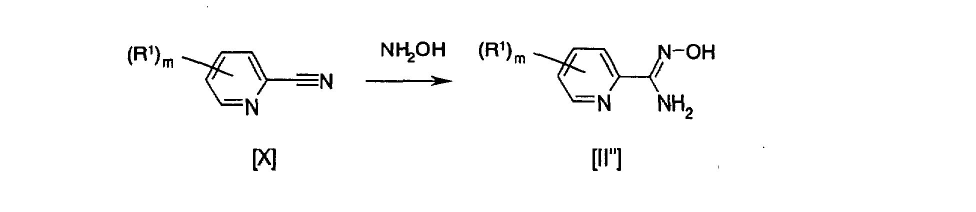 (wherein, R¹ and m are as defined above.)

A compound of Formula [II''] can be produced by reaction of a 2-cyanopyridine with hydroxylamine.

Among the compounds of Formula [II], which are starting materials for producing the compounds of the present invention, a compound represented by Formula [III''] where R² is cyano can be synthesized according to the following method: 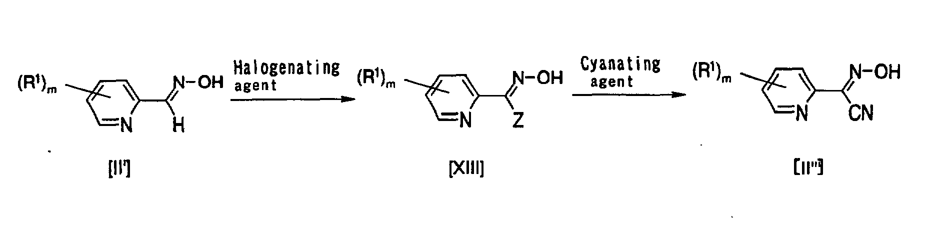 (wherein, R¹, Z and m are as defined above.)

A compound of Formula [II"'] can be produced by reacting a 2-pyridinecarboxyaldoxime of Formula [II'] with a halogenating agent, such as chlorine gas, N-chlorosuccinimide (NSC) or N-bromosuccinimide (NBS), to give a compound of Formula [XIII], followed by a reaction with a cyanating agent, such as NaCN or KCN.

Among the compounds of Formula [III], which are starting materials for producing the compounds of the present invention, a heterocyclic compound represented by Formula [III'] where R³ and R⁴ are hydrogen and L is halogen can be synthesized according to the following method: 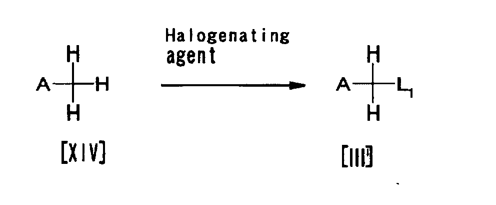 (wherein, A is as defined above, and L₁ is halogen such as chlorine, bromine or iodine.)

A heterocyclic compound having a halomethyl group and represented by Formula [III'] can be synthesized by reacting a heterocyclic compound having a methyl group and represented by Formula [XIV] is with a halogenated succinimide, such as NCS, NBS or N-iodosuccinimide (NIS), under irradiation of light.

A heterocyclic compound with a halomethyl or sulfonyloxymethyl group and represented by Formula [III'] can also be synthesized according to the following method: 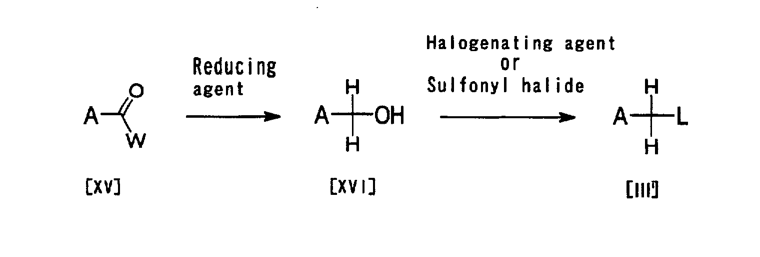 (wherein, A and L are as defined above and W is halogen such as chlorine, hydroxyl, hydrogen or lower alkoxy.)

To produce a compound of Formula [III'], a heterocyclic compound of Formula [XV] is reacted with an appropriate reducing agent, such as LiAlH₄ or NaBH₄, to give a heterocyclic compound with a hydroxymethyl group and represented by Formula [XVI], followed by halogenation of the hydroxyl group with a halogenating agent, such as thionyl chloride, to a halogen atom, or by reacting with a sulfonyl halide, such as methane sulfonyl chloride, to derive to an oxysulfonyl group.

Among the compounds of Formula [III], which are starting materials for producing the compounds of the present invention, a heterocyclic compound represented by Formula [III''] where R³ and/or R⁴ are C₁₋₆ alkyl, C₃₋₆ cycloalkyl or C₁₋₆ haloalkyl can be synthesized according to the following method: 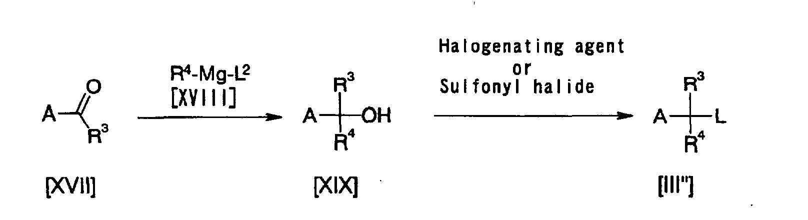 (wherein, A and L are as defined above, and R³ and/or R⁴ are C₁₋₆ alkyl, C₃₋₆ cycloalkyl or C₁₋₆ haloalkyl; and L² is a leaving group including halogen such as chlorine, bromine or iodine.)

To produce a compound of Formula [III''], a ketone of Formula [XVII] is reacted with a Grignard reagent of Formula [XVIII] to derive a methyl alcohol of Formula [XIX], followed by halogenation of the hydroxyl group with a halogenating agent, such as thionyl chloride, to a halogen atom, or by reacting with a sulfonyl halide, such as methane sulfonyl chloride, to change to an oxysulfonyl group.

### Process 2:

Of the compounds of the present invention, a compound where R² is hydrogen, C₁₋₆ alkyl, C₃₋₆ cycloalkyl or C₁₋₆ haloalkyl can also be produced according to the following method: 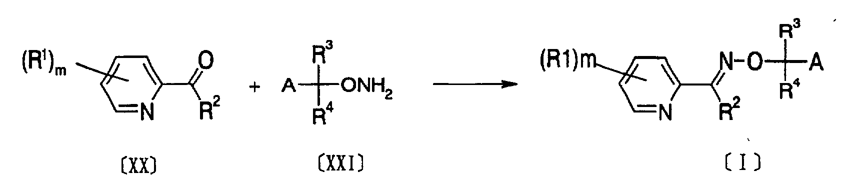 (wherein, R¹, R³ , R⁴ and A are as defined above, and R² is hydrogen, C₁₋₆ alkyl, C₃₋₆ cycloalkyl or C₁₋₆ haloalkyl.)

A compound of Formula [I] can be produced by stirring a compound of Formula [XX] with a compound of Formula [XXI] or its salt, without a solvent or preferably in a solvent, at a reaction temperature of 0 to 150°C for 10 minutes to 24 hours.

Examples of suitable solvents for use include alcohols such as ethanol and methanol; ethers such as diethyl ether, THF and dioxane; cellosolves such as methyl cellosolve and ethyl cellosolve; aromatic hydrocarbons such as benzene and toluene; acetic acid; water; amides such as DMF and N,N-dimethylacetamide; and dimethyl sulfoxide. These can be used alone or as a mixed solvent of two or more at various mixing ratios.

A catalyst is not indispensable in the reaction. An addition of an acid or base may greatly accelerate the reaction in some cases. Examples of suitable acids for use include inorganic acids such as sulfuric acid and hydrochloric acid; and organic acids such as paratoluenesulfonic acid. Sodium acetate is exemplified as a base. 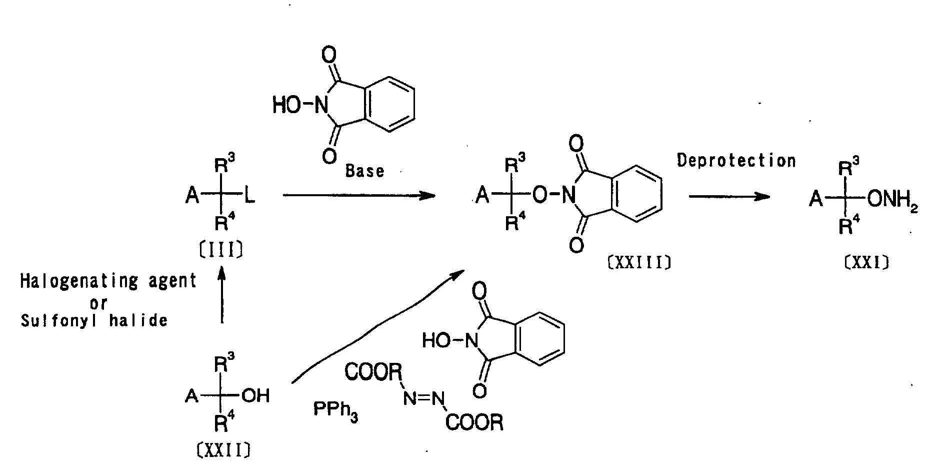 (wherein, A, R³, R⁴, R and L are as defined above.)

Oxyamines of Formula [XXI], which are starting materials for producing the compounds of the present invention, can be produced by reacting a compound represented by Formula [III] is with N-hydroxyphthalimide in an appropriate solvent in the presence of a proper base, to derive a compound of Formula [XXIII], followed by deprotection with a deprotecting agent such as hydrazine.

A compound of Formula [XXIII] may also be synthesized by reacting an alcohol of Formula [XXII] with N-hydroxyphthalimide in an appropriate solvent in the presence of triphenylphosphine and diethyl azodicarboxylate (DEAD).

To produce salts of compounds of Formula [I], a compound of Formula [I] is reacted with an inorganic or organic acid in an appropriate solvent.

Upon completion of the reaction, ordinary posttreatments give the target compound. There exist isomers of the compounds of the present invention at the oxime moiety. These isomers are all covered by the invention. The structures of the compounds of the present invention are determined by NMR, mass spectroscopy and other means.

Representative examples of the compounds of the present invention that are produced according to the methods mentioned above are shown in Table 1. The abbreviations and symbols in the table stand for the following:

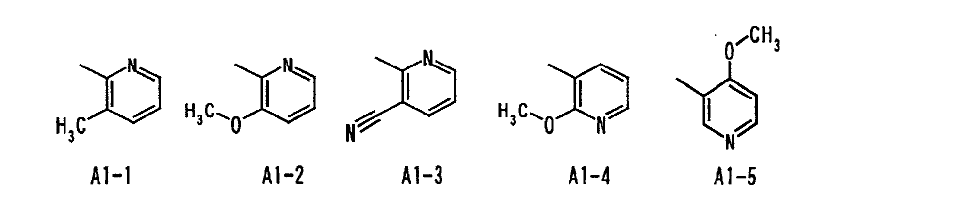

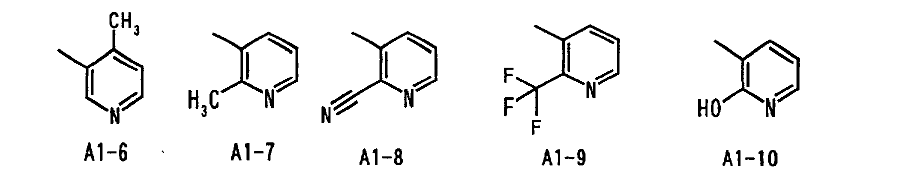

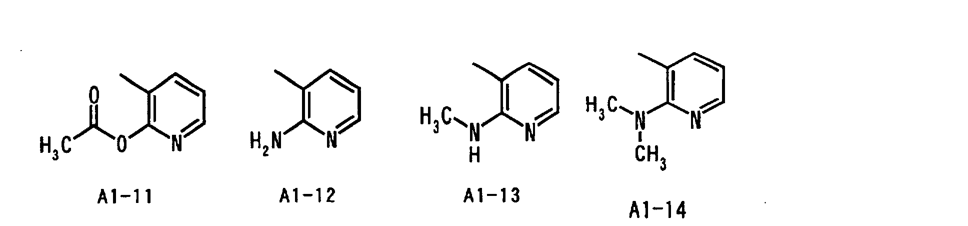

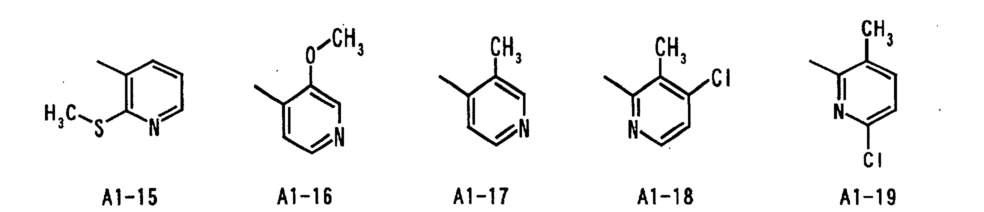

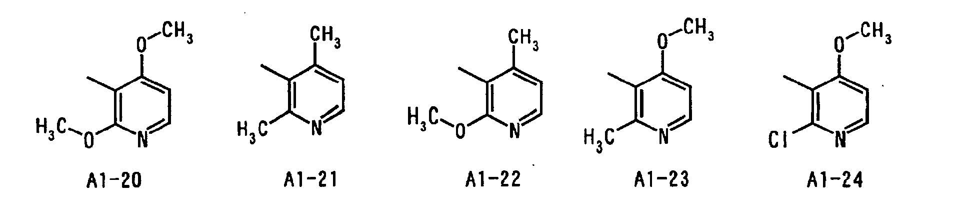

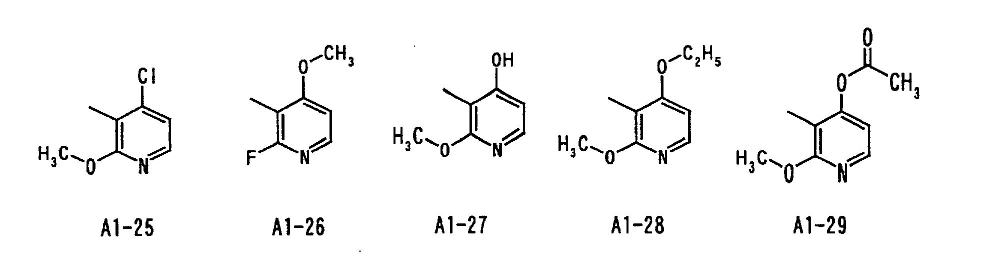

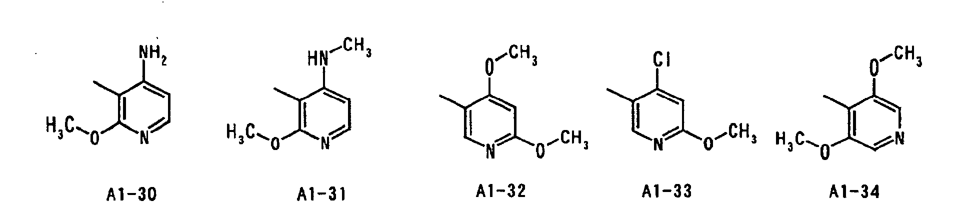

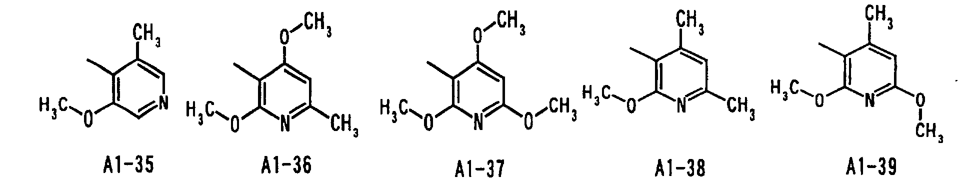

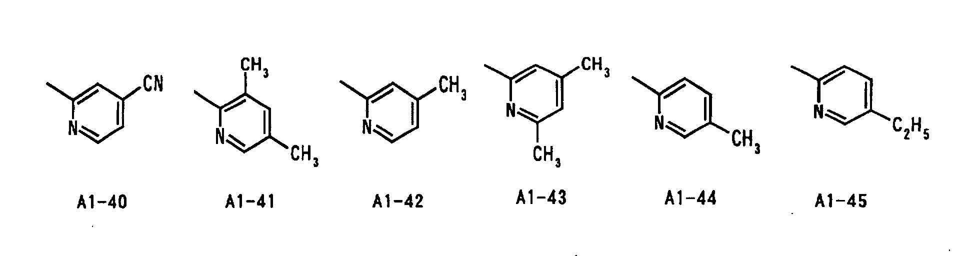

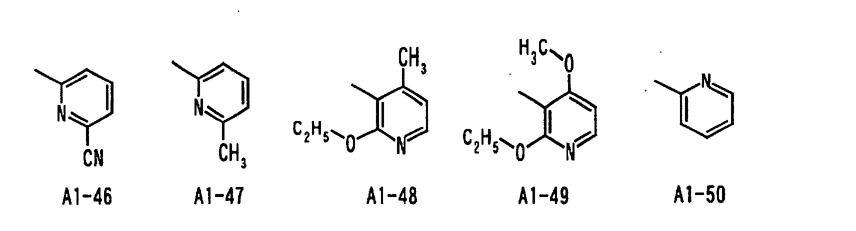

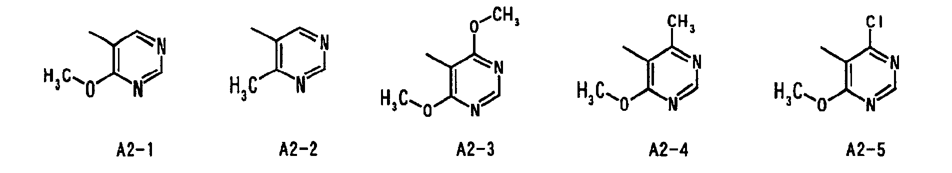

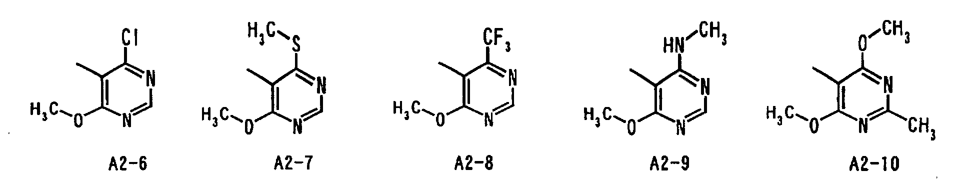

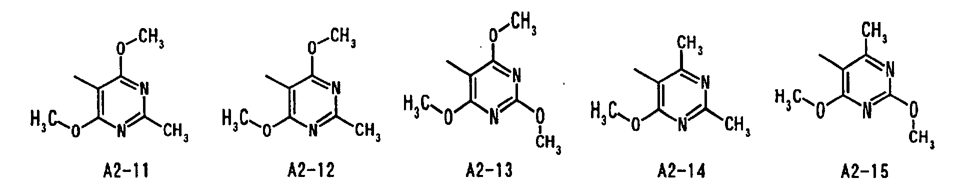

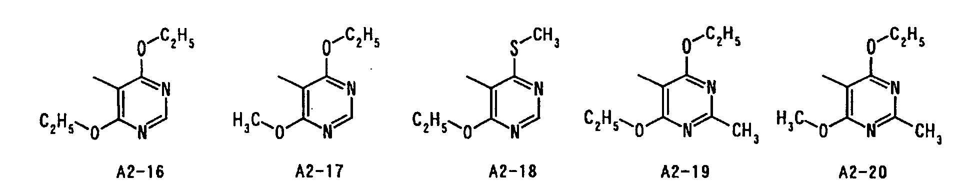

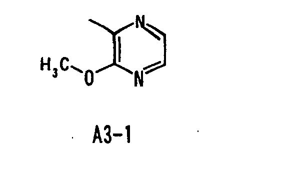

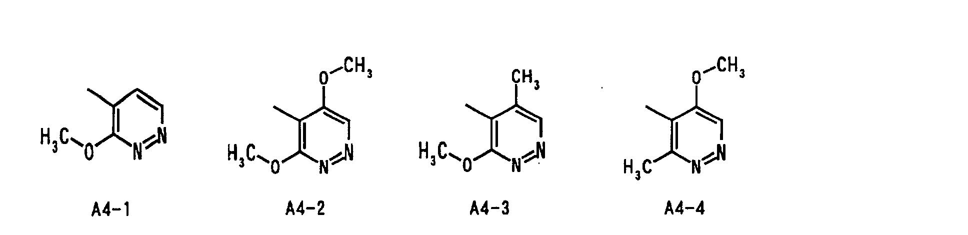

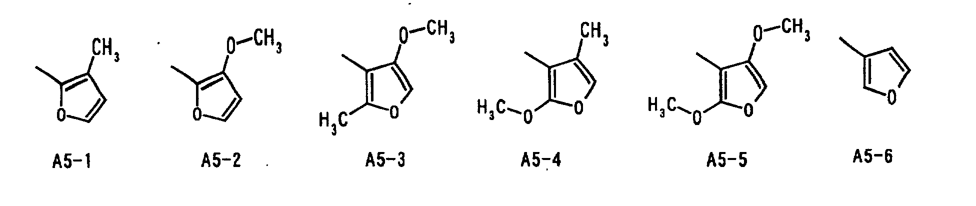

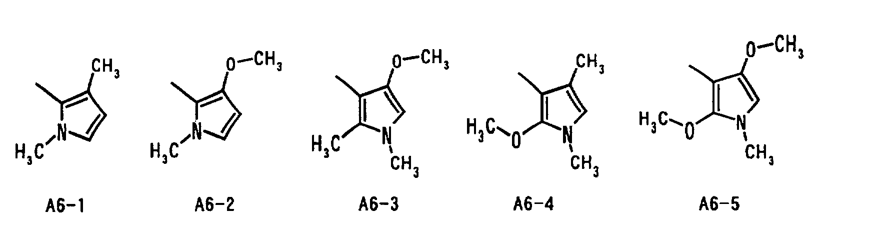

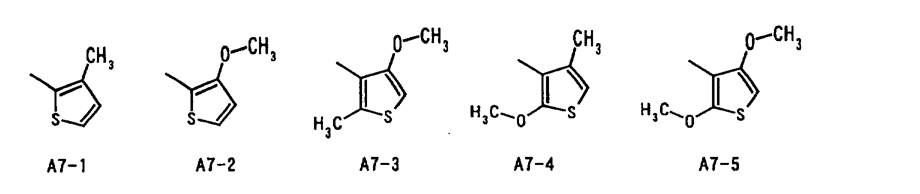

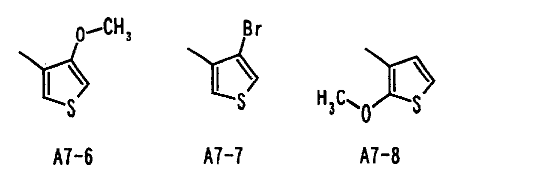

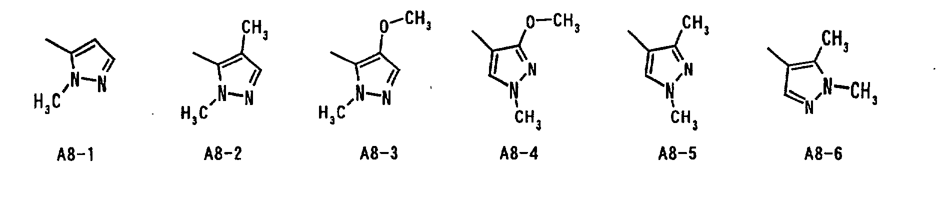

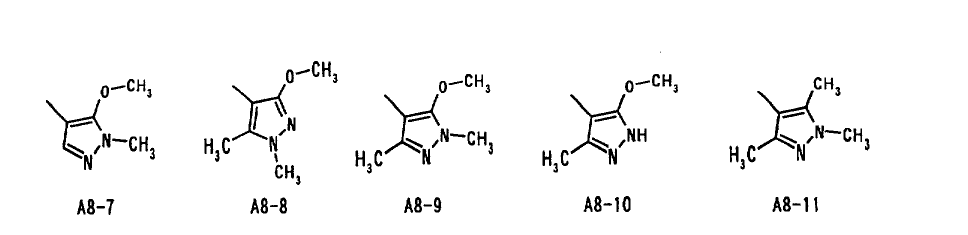

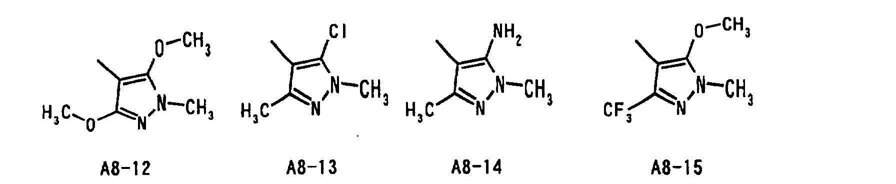

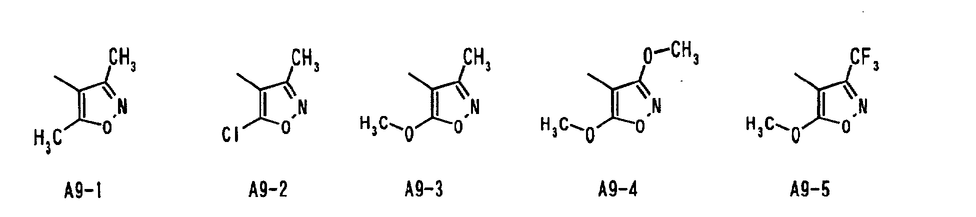

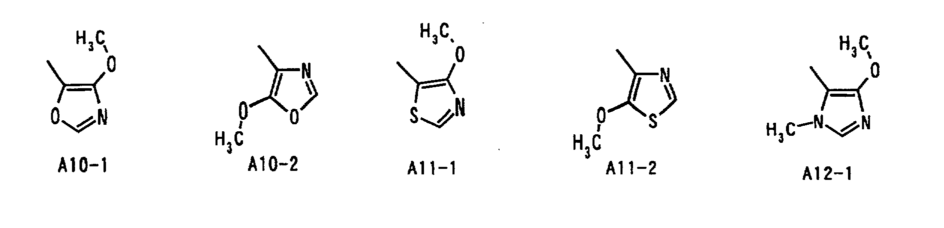

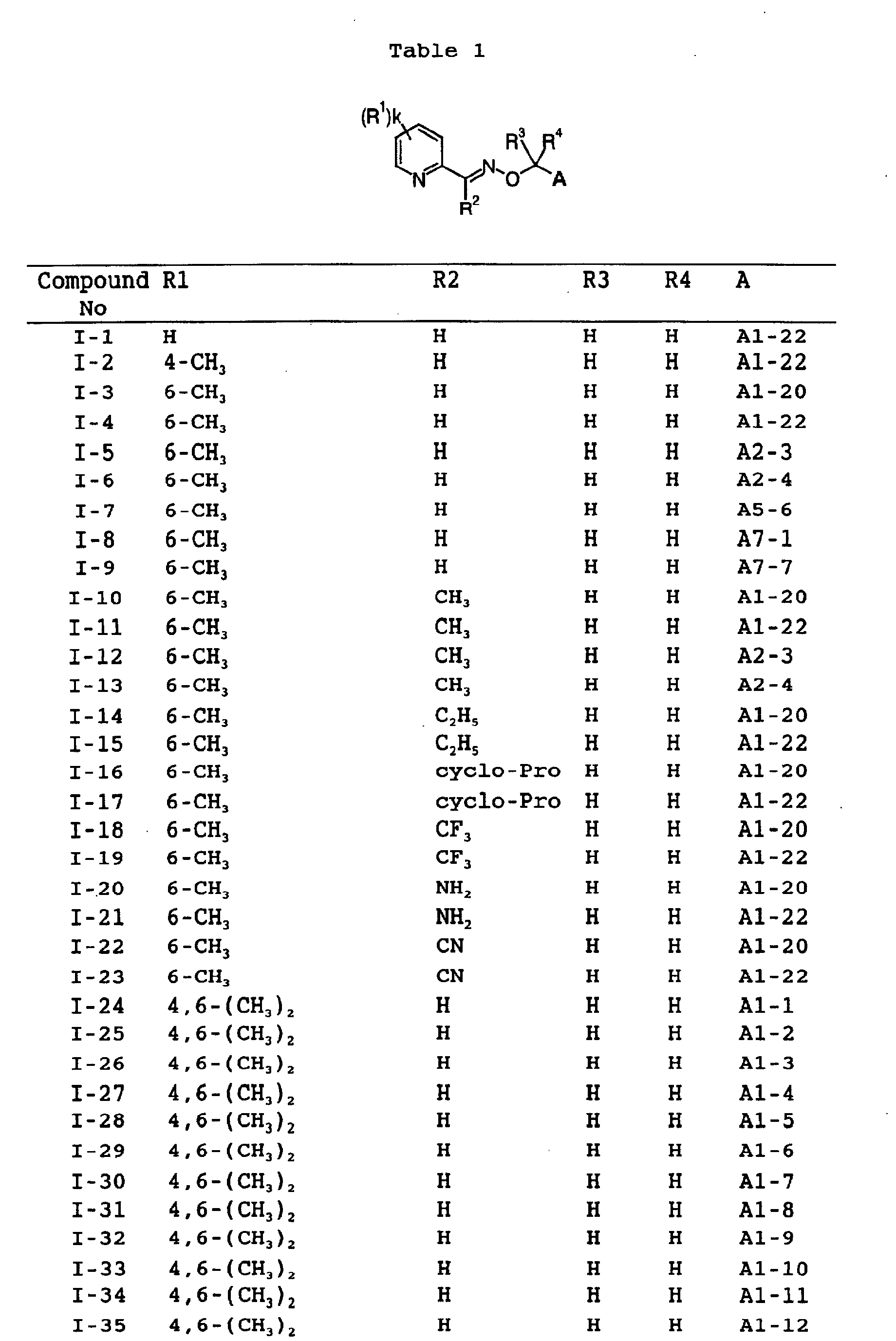

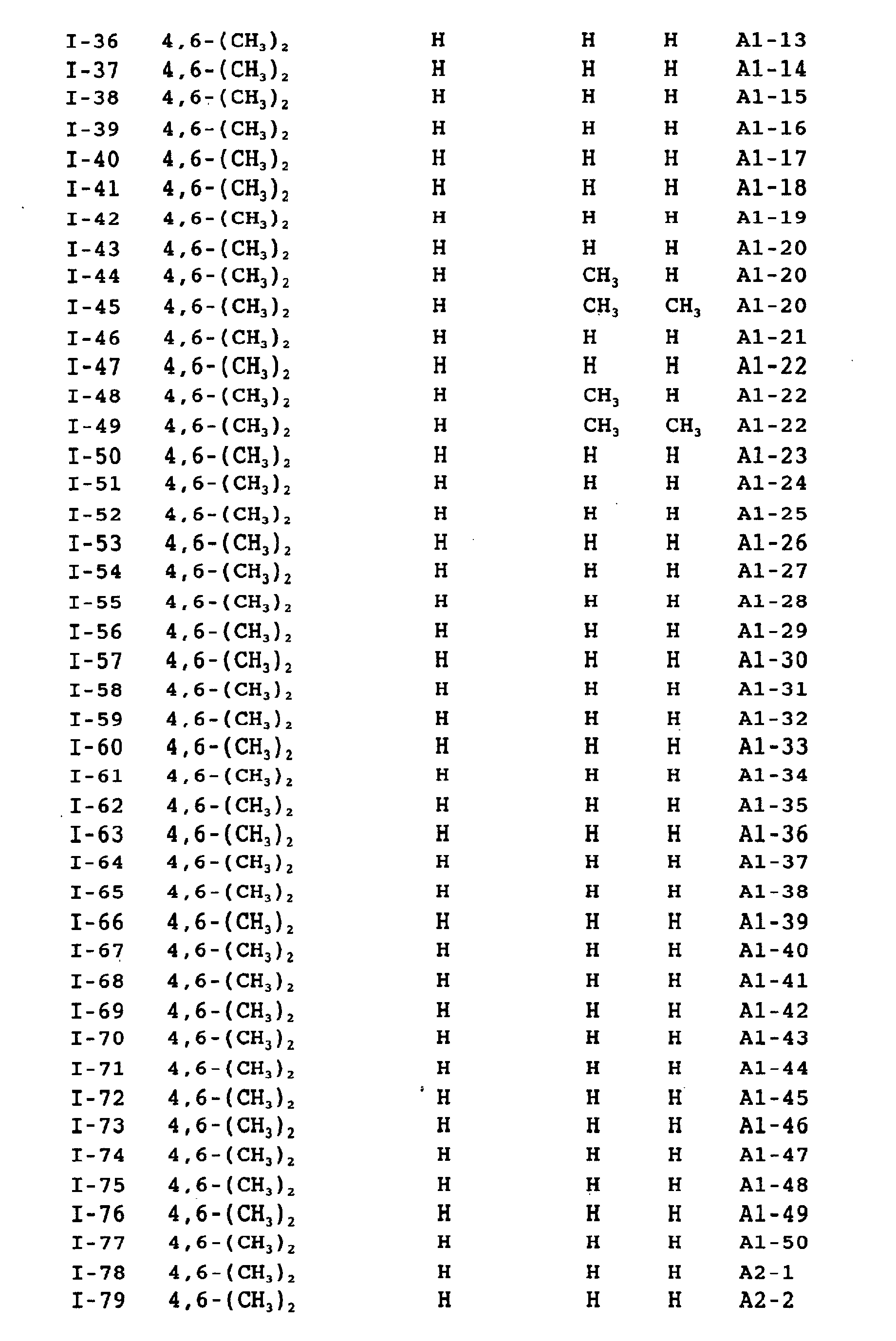

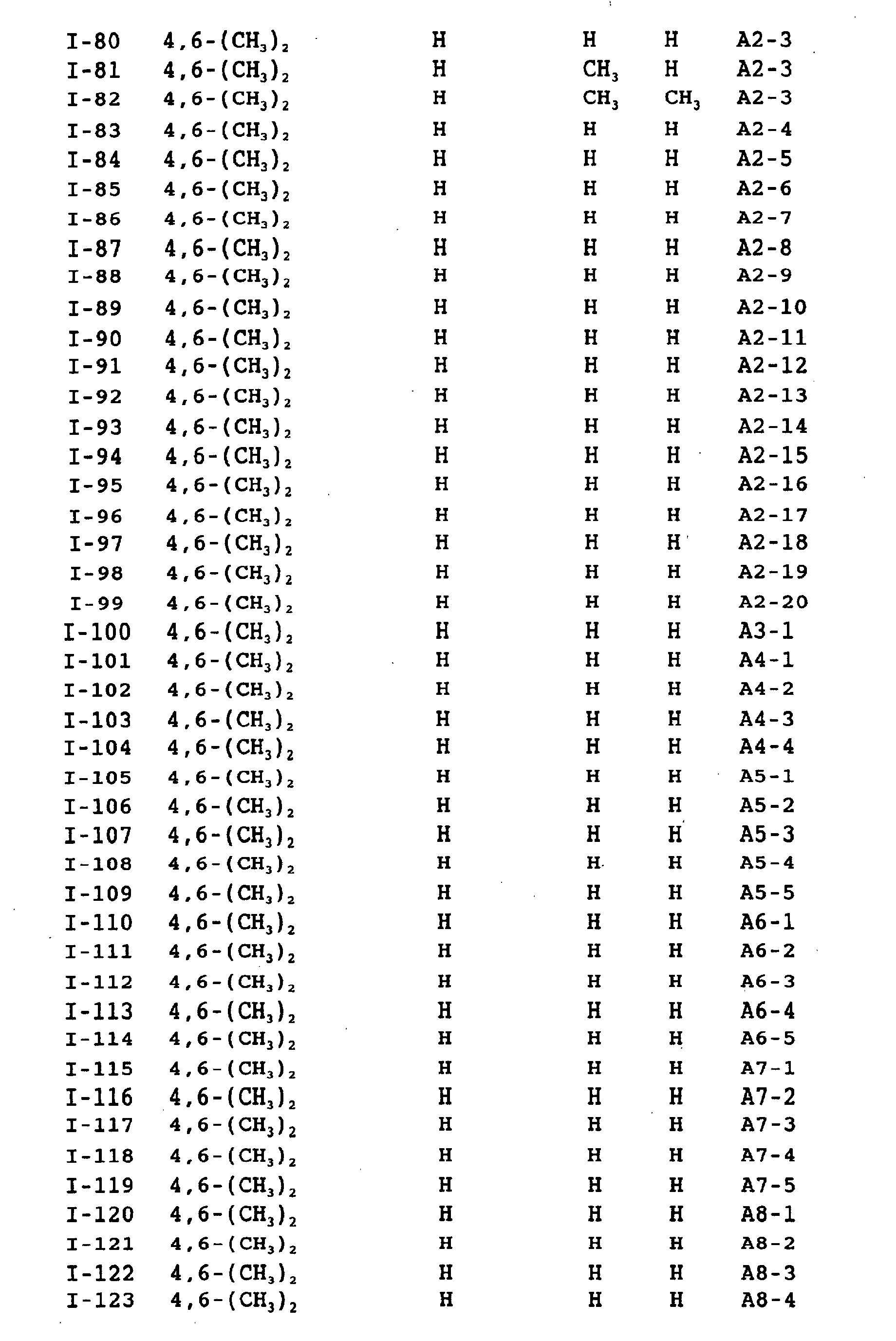

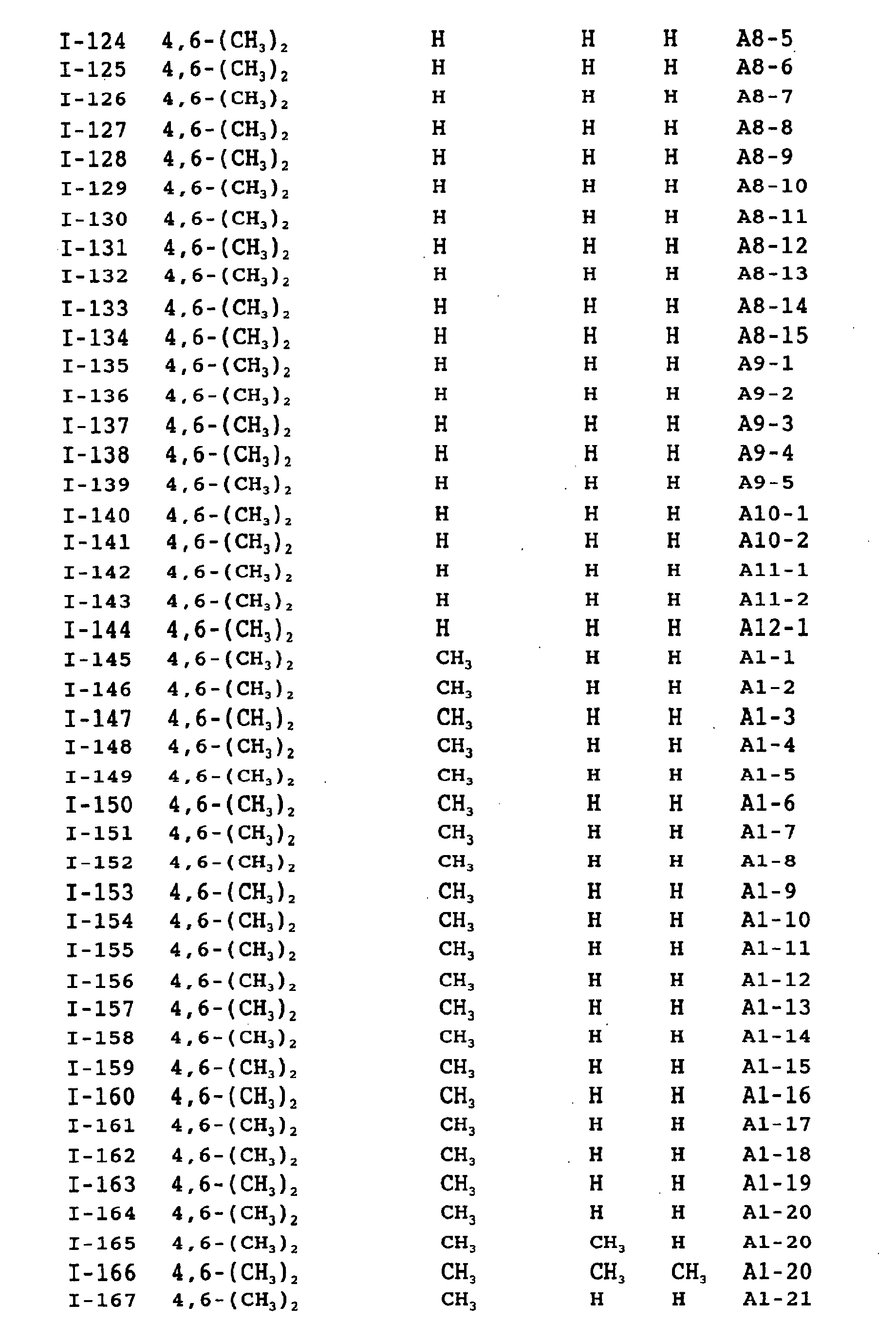

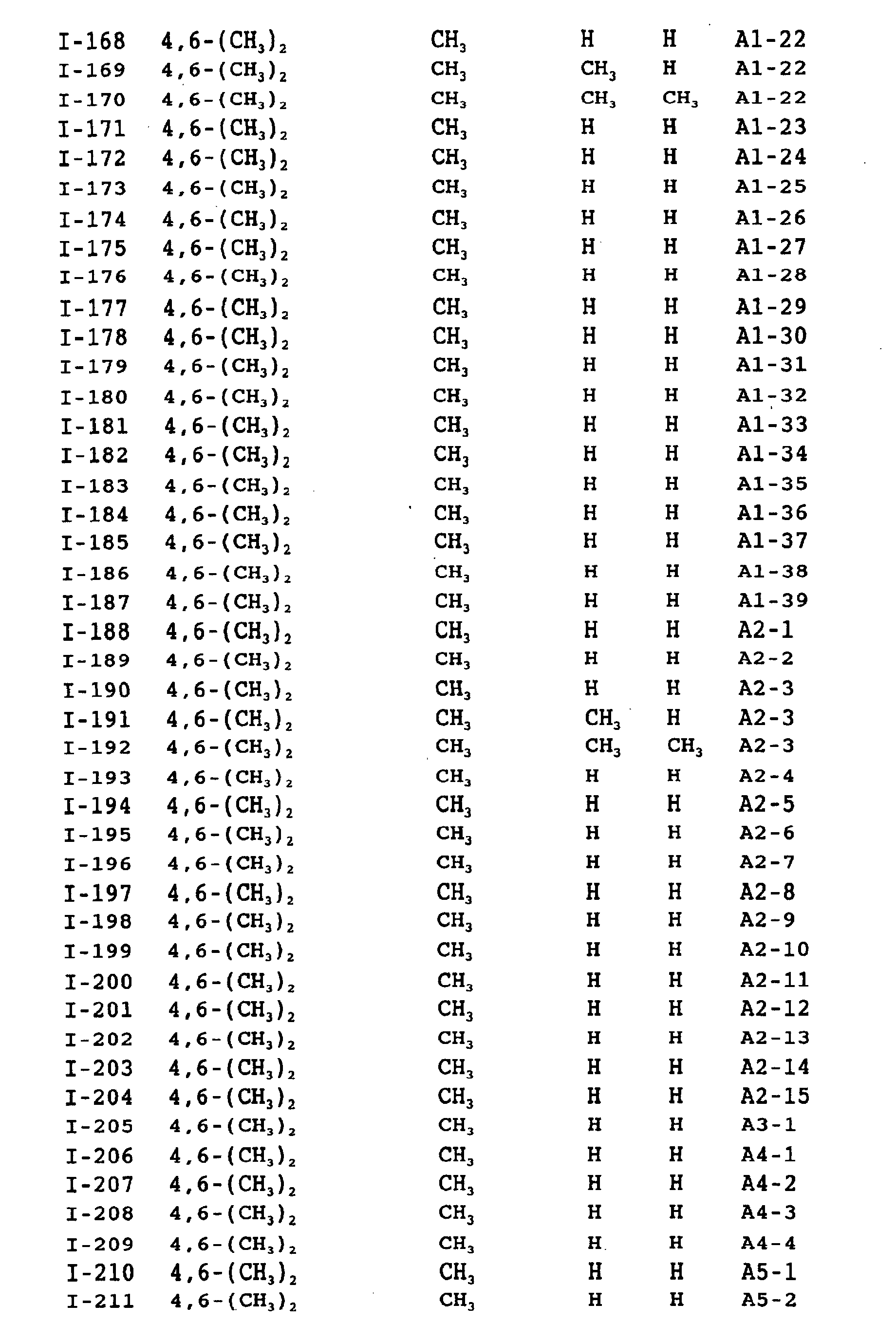

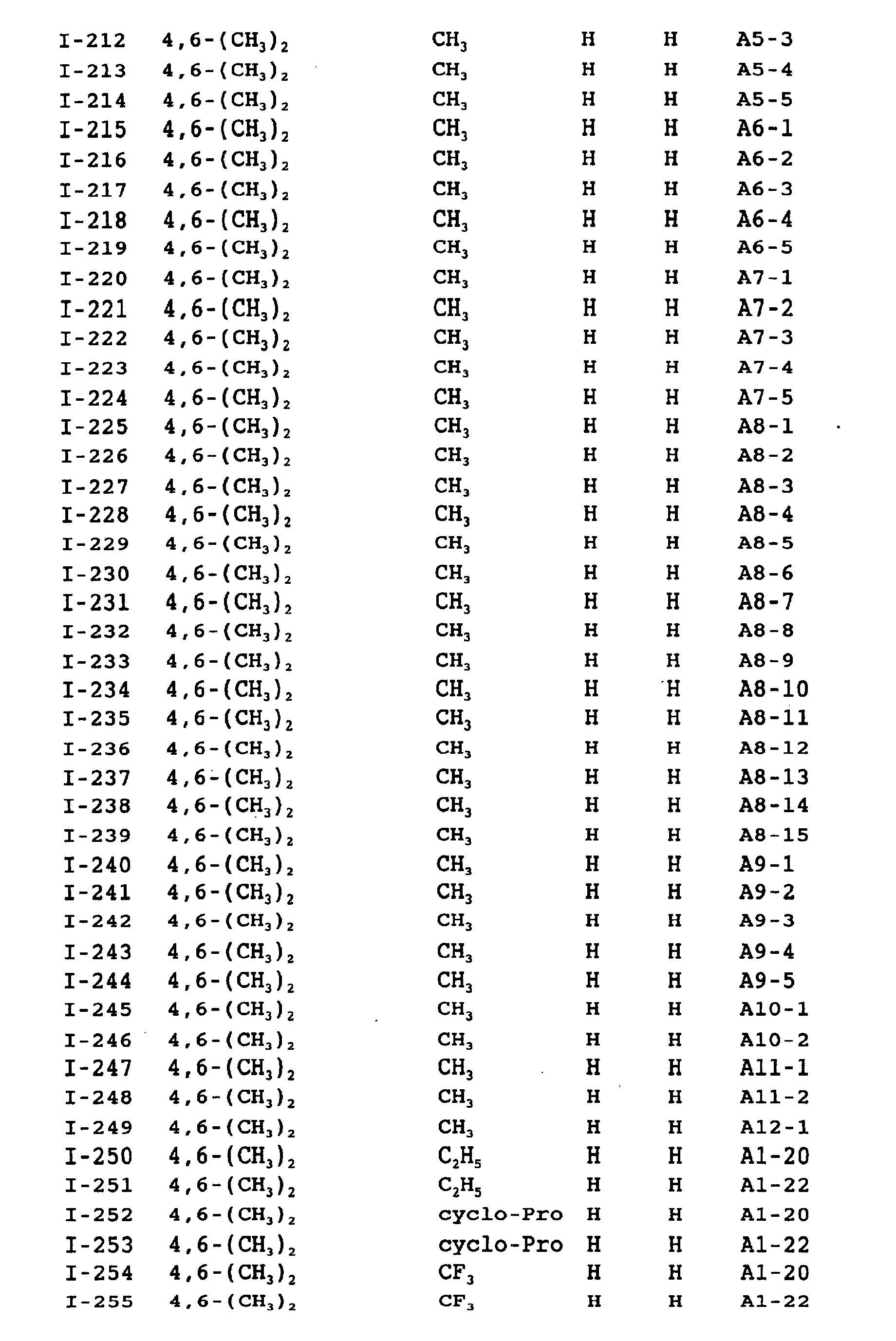

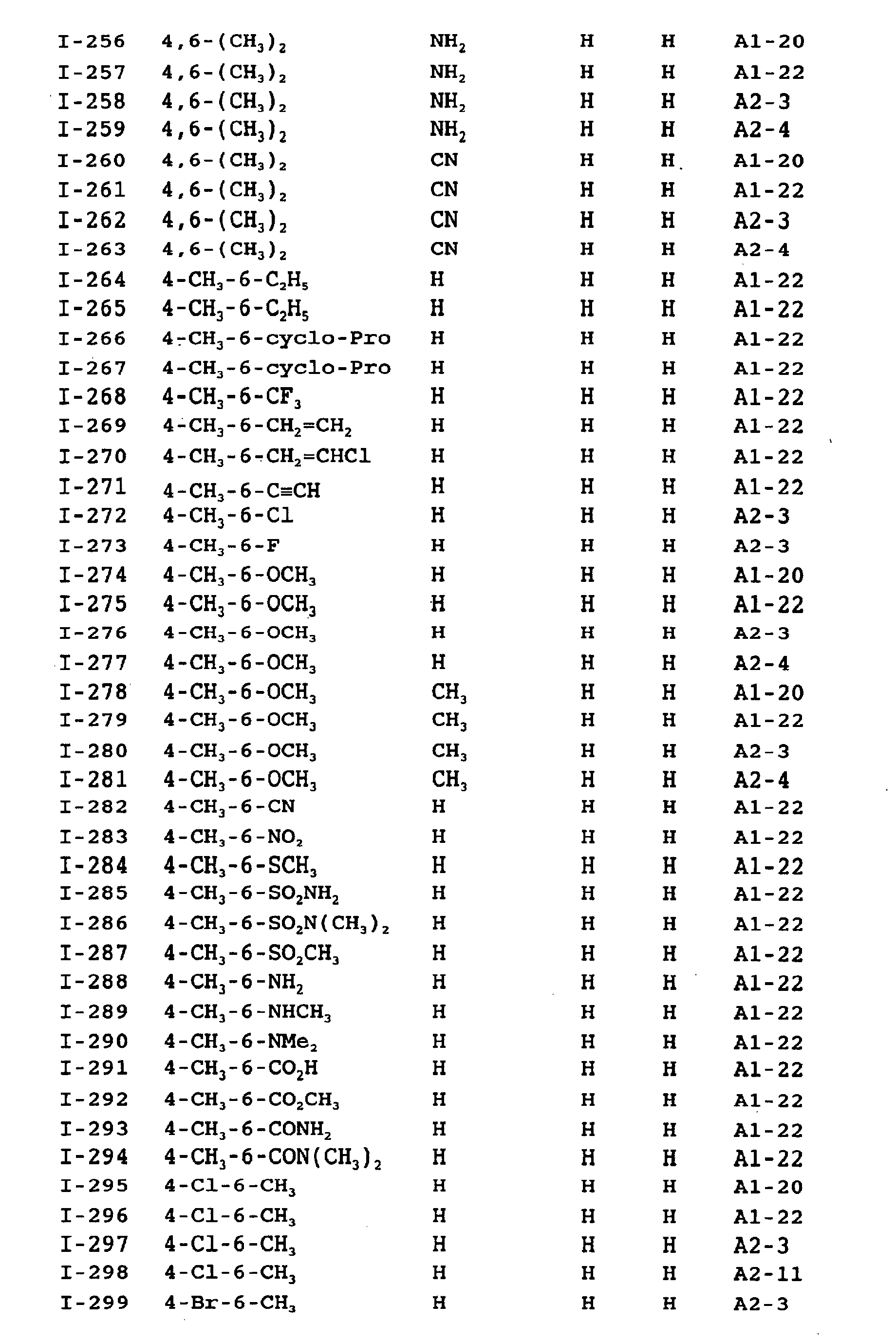

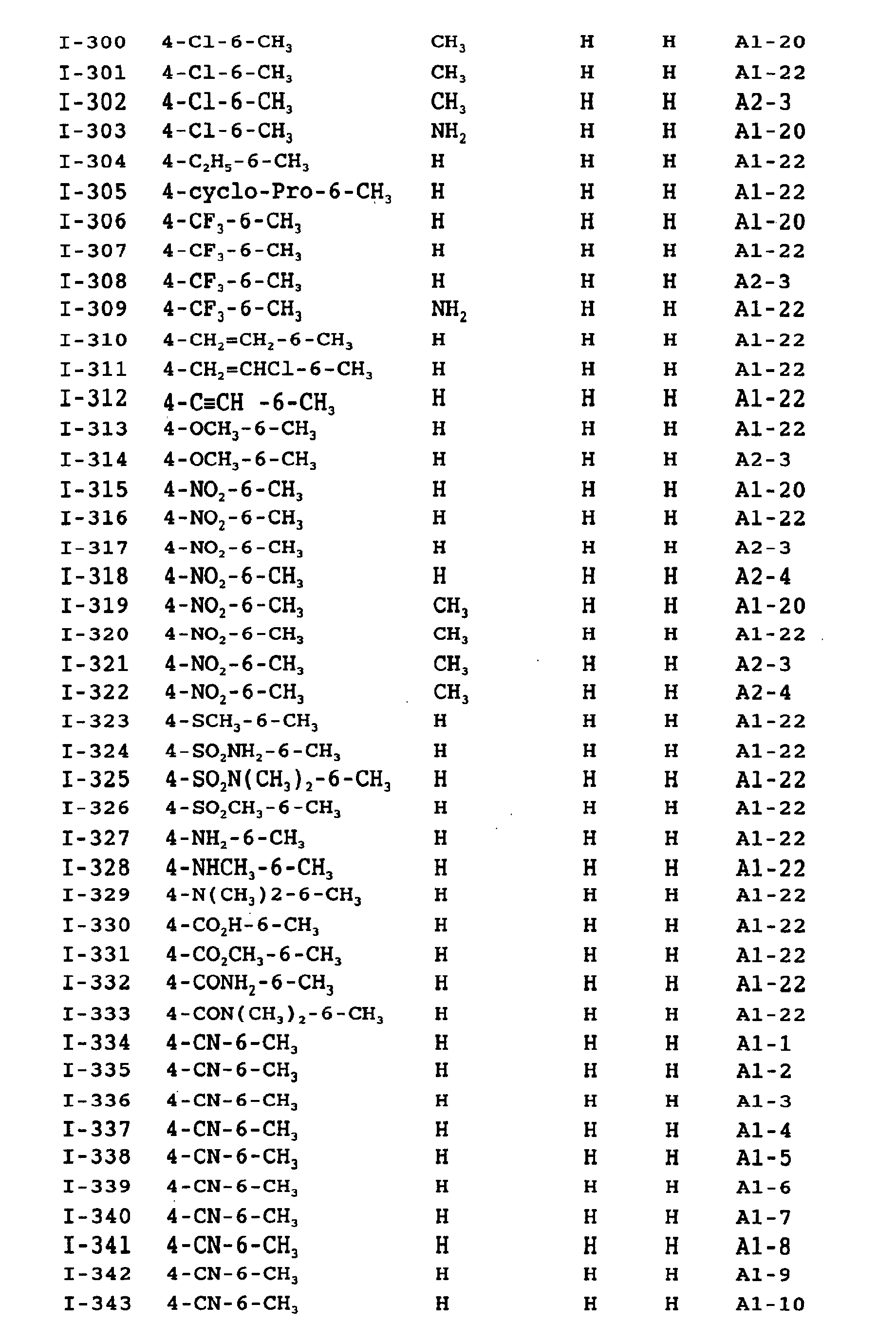

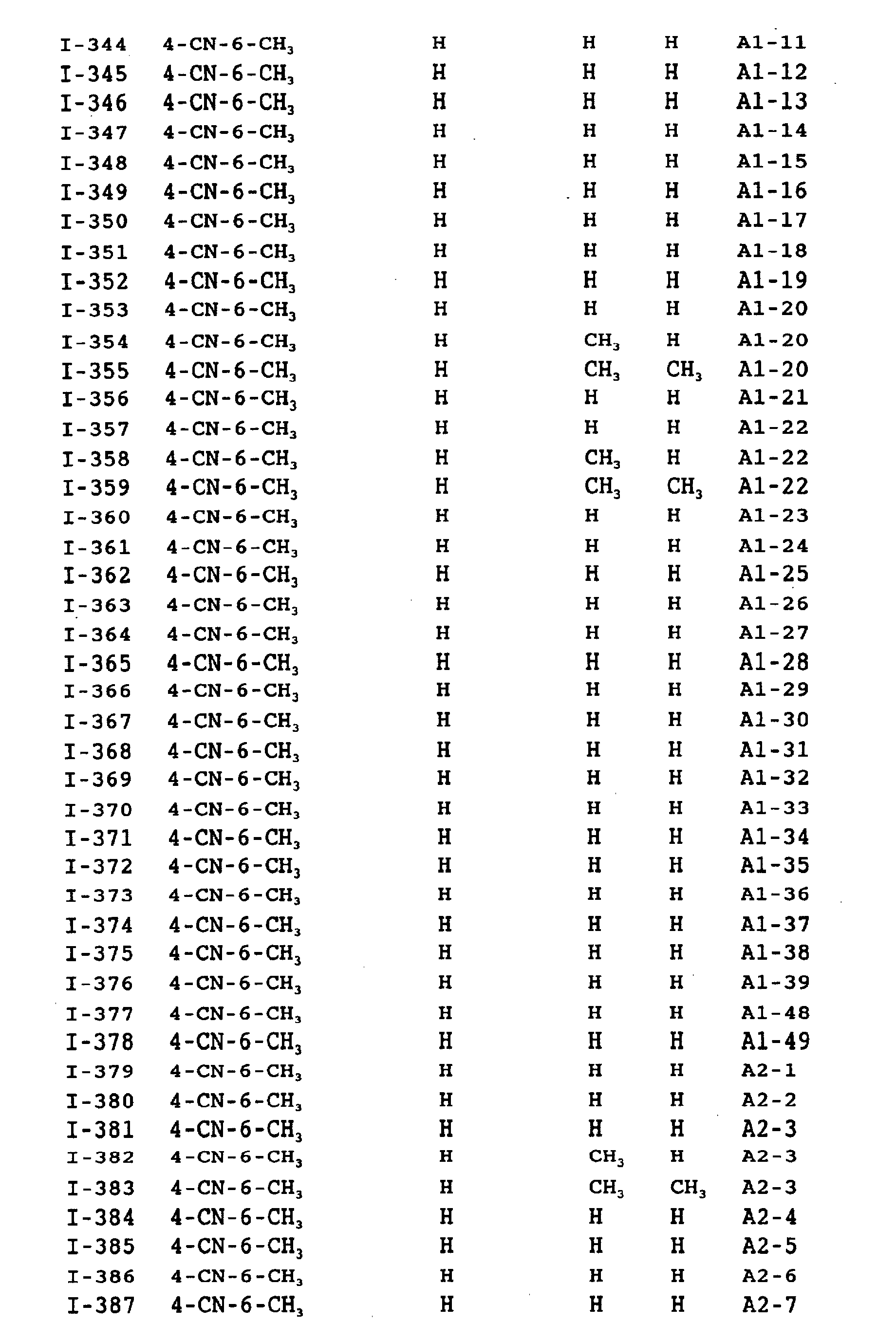

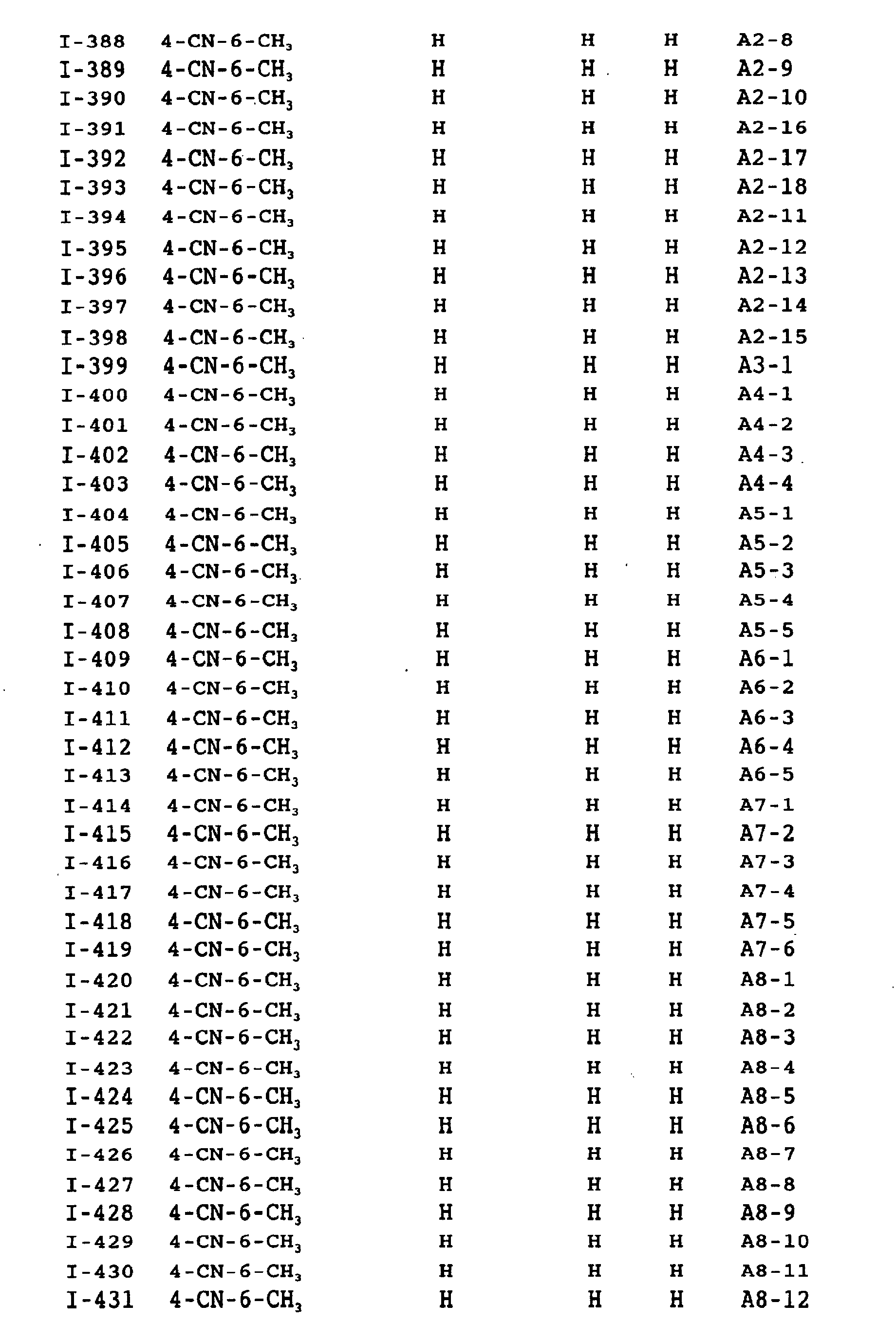

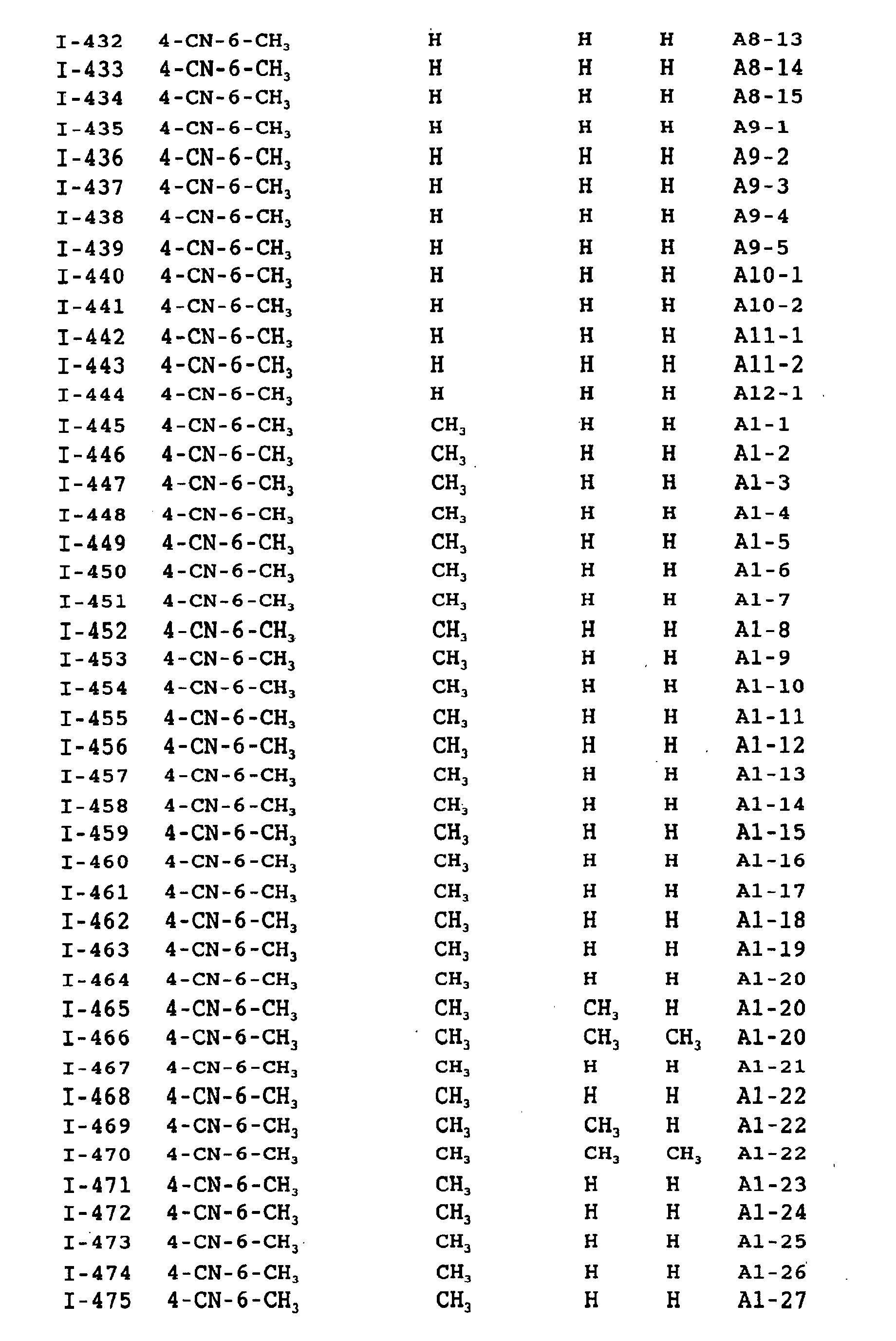

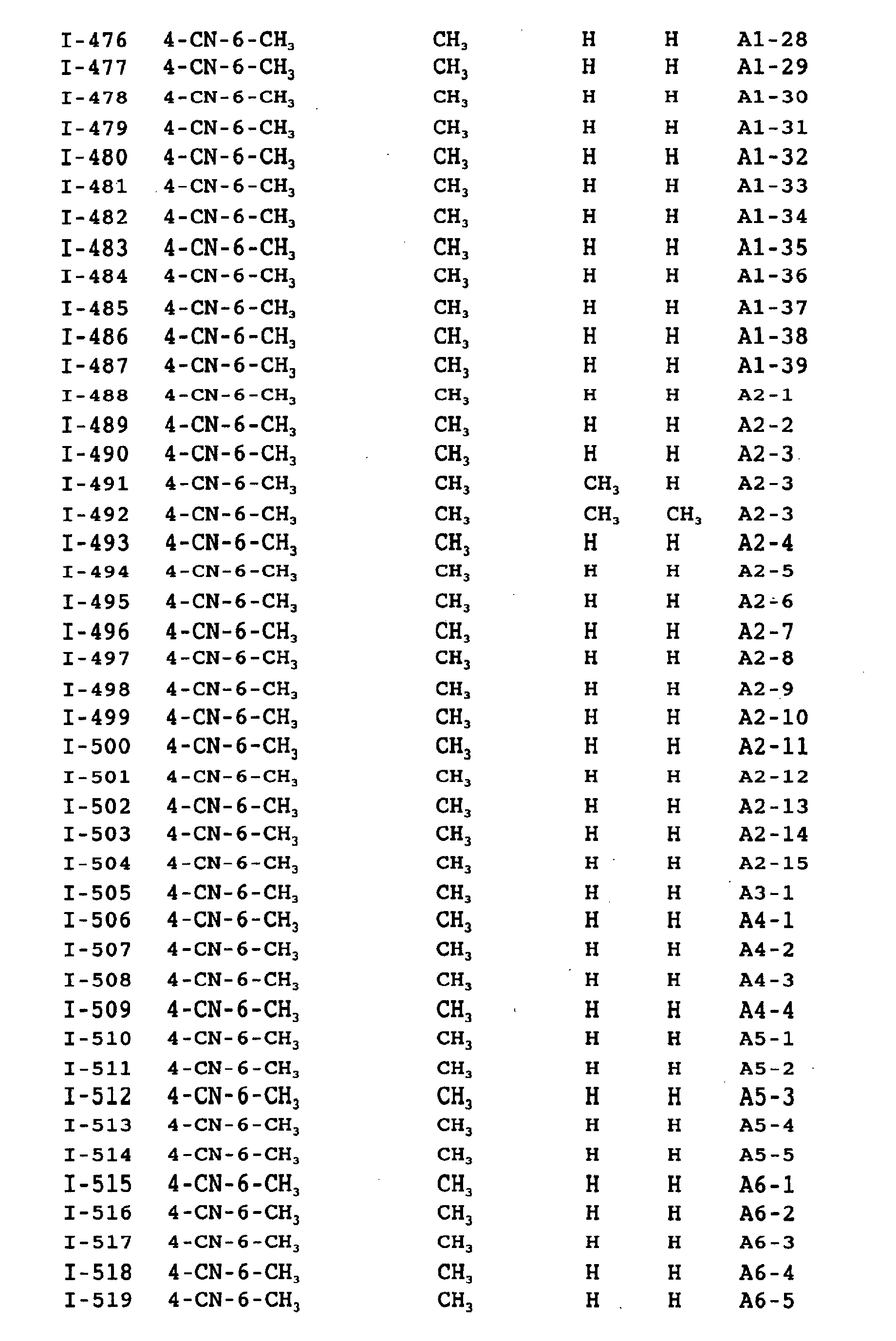

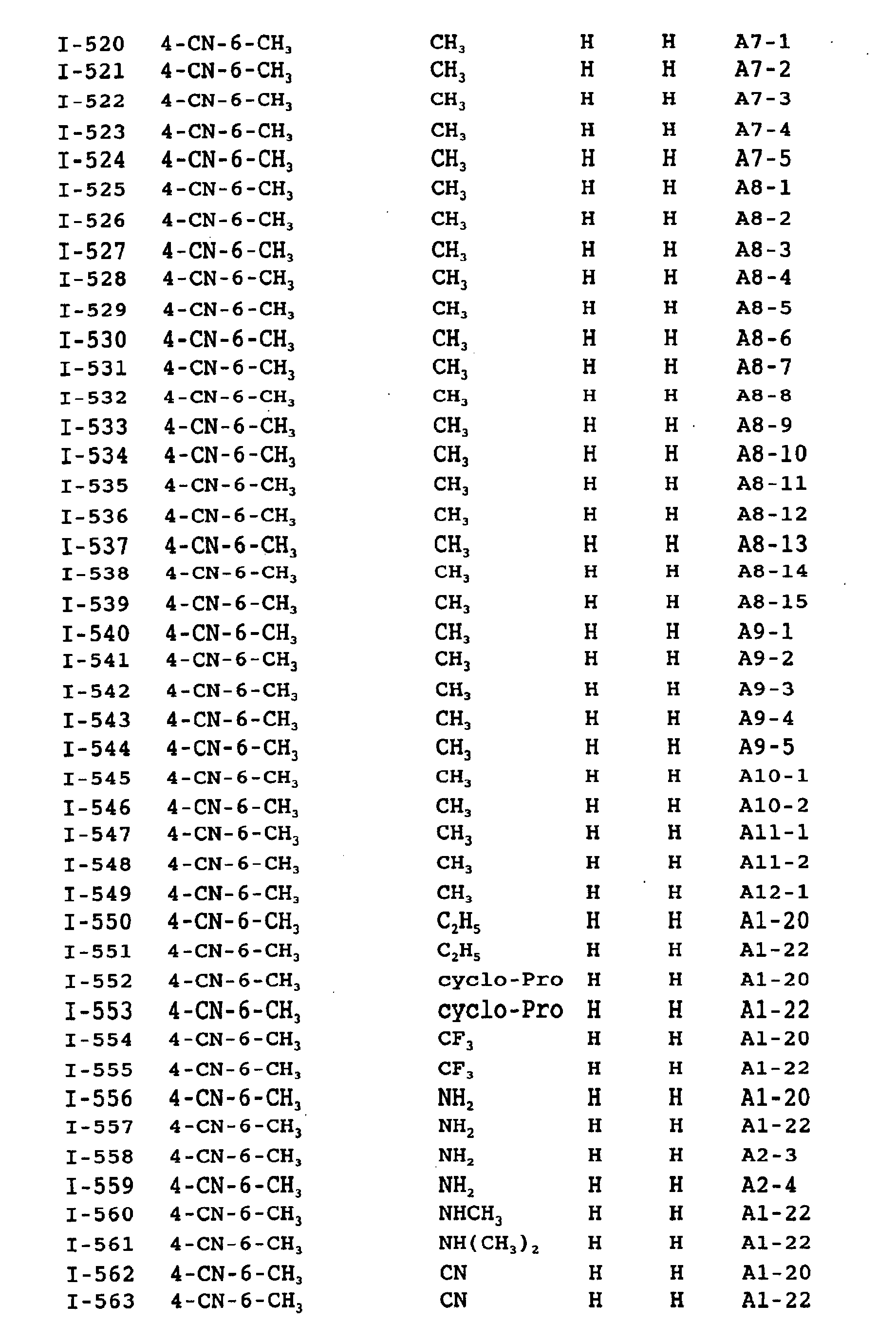

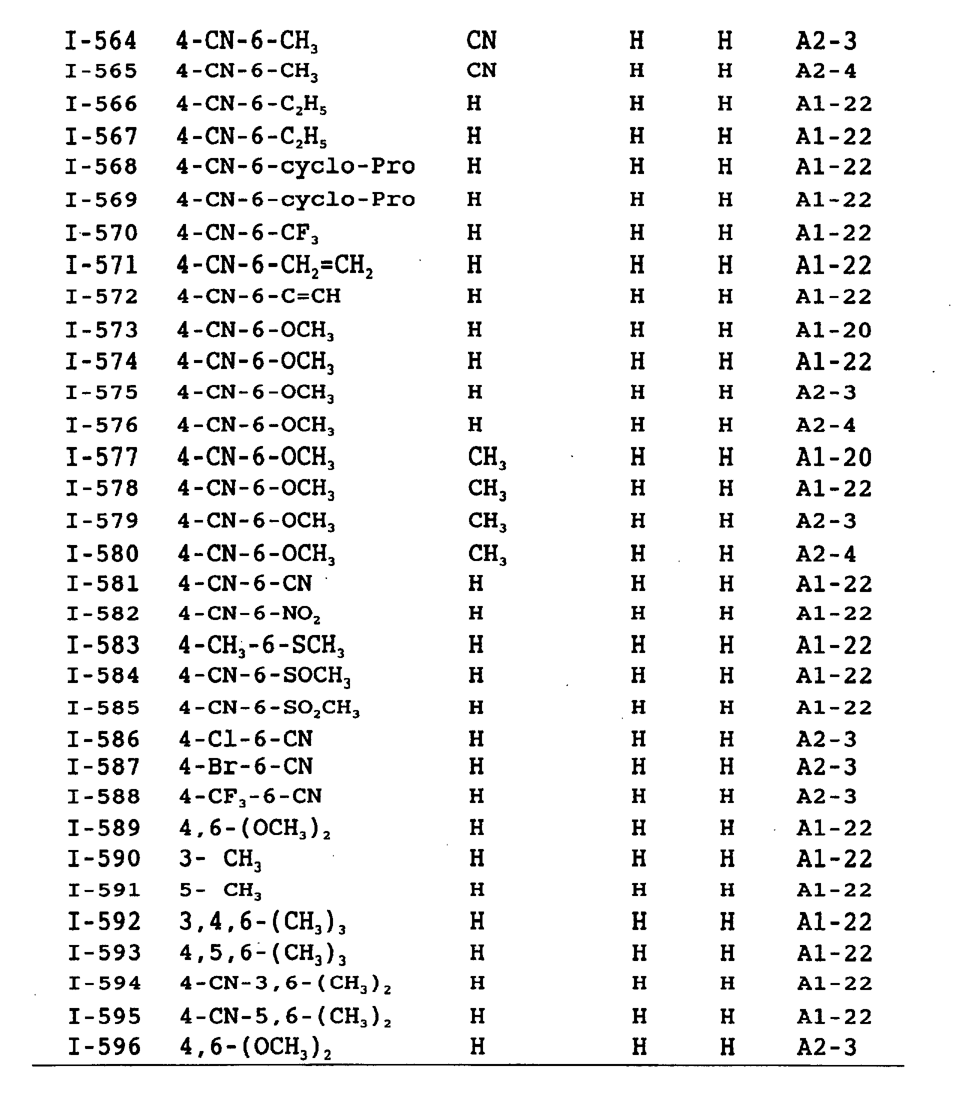

### BEST MODE FOR CARRYING OUT THE INVENTION:

The present invention is described in detail with reference to Examples.

### Example 1

### Preparation of 4,6-dimethyl-2-pyridinacarboxyaldehyde O-[(2-methoxy-4-methyl-3-pyridinyl)methyl]oxime (Compound No. II-3)

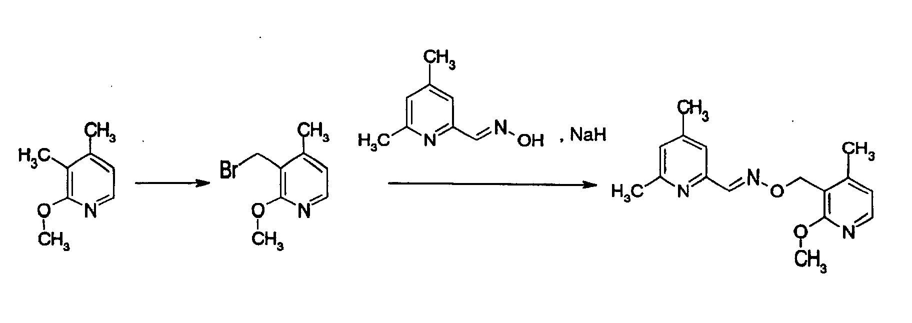

To a solution of 0.95 g (6.93 mmol) of 3,4-dimethyl-2-methoxypyridine in 10 ml of carbon tetrachloride was added 1.25 g (7.02 mmol) of N-bromosuccinimide. . The resulting solution was irradiated with light (infrared light 375 WR, produced by Toshiba Co., Ltd.) for an hour at the refluxing temperature. The reaction solution was cooled to room temperature. The deposited succinimide was separated by filtration. The filtrate was concentrated under reduced pressure to give a crude product of 3-bromomethyl-2-methoxy-4-methylpyridine.

Meanwhile, to a solution of 1.05 g (7.00 mmol) of 4,6-dimethylpyridine-2-carboxyaldehyde oxime in 10 ml of N,N-dimethylformamide was added 0.28 g (7.00 mmol) of sodium hydride (oilniess: 60%) while cooling in an ice bath, and the solution was stirred at the ice temperature for 30 minutes. To the resulting solution was added the whole amount of the previously prepared crude product of 3-bromomethyl-2-methoxy-4-methylpyridine while cooling in an ice bath, and the solution was stirred at room temperature for an hour. The reaction solution was poured into ice-water, and extracted with diethyl ether. The organic layer was washed with water, dried over anhydrous magnesium sulfate and concentrated under reduced pressure. The obtained crude product was purified by silica gel column chromatography [eluted with n-hexane:ethyl acetate = 7:3 (v/v)] to give 1.70 g of the target compound.
Melting point: 54 to 55°C.

### Example 2

### Preparation of 1-(4,6-dimethyl-2-pyridinyl)ethanone O-[(2,4-dimethoxy-3-pyridinyl)methyl]oxime (Compound No. II-14)

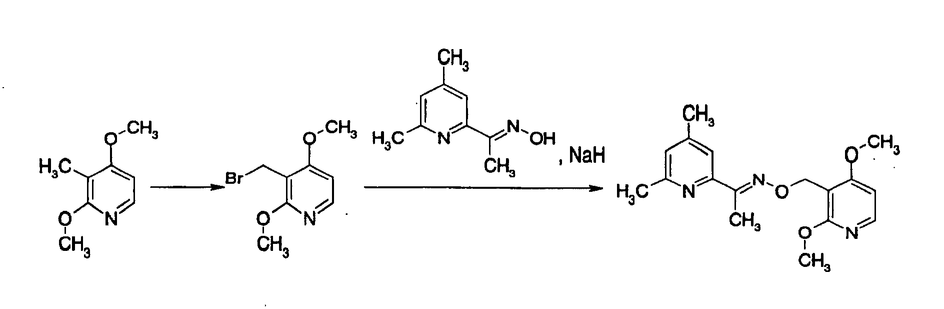

To a solution of 0.50 g (3.27 mmol) of 2,4-dimethoxy-3-methylpyridine in 15 ml of carbon tetrachloride was added 0.58 g (3.26 mmol) of N-bromosuccinimide. . The resulting solution was irradiated with light (infrared light 375 WR, produced by Toshiba Co., Ltd.) for an hour at the refluxing temperature. The reaction solution was cooled to room temperature. The deposited succinimide was separated by filtration. The filtrate was concentrated under reduced pressure to give a crude product of 3-bromomethyl-2,4-dimethoxypyridine.

Meanwhile, to a solution of 0.43 g (2.62 mmol) of 1-(4,6-dimethyl-2-pyridinyl)ethanone oxime in 10 ml of N,N-dimethylformamide was added 0.13 g (3.25 mmol) of sodium hydride (oiliness: 60%) while cooling in an ice bath, and the solution was stirred at the ice temperature for 30 minutes. To the resulting solution was added the whole amount of the previously prepared crude product of 3-bromomethyl-2,4-dimethoxypyridine while cooling in an ice bath, and the solution was stirred at room temperature for an hour. The reaction solution was poured into ice-water, and extracted with diethyl ether. The organic layer was washed with water, dried over anhydrous magnesium sulfate and concentrated under reduced pressure. The obtained crude product was purified by silica gel column chromatography [eluted with n-hexane:ethyl acetate = 7:3 (v/v)] to give 0.54 g of the target compound. Melting point: 114 to 116°C.

### Example 3

### Preparation of 1-(4,6-dimethyl-2-pyridinyl)ethanone O-[(4,6-dimethoxy-5-pyrimidinyl)methyl]oxime (Compound No. II-17)

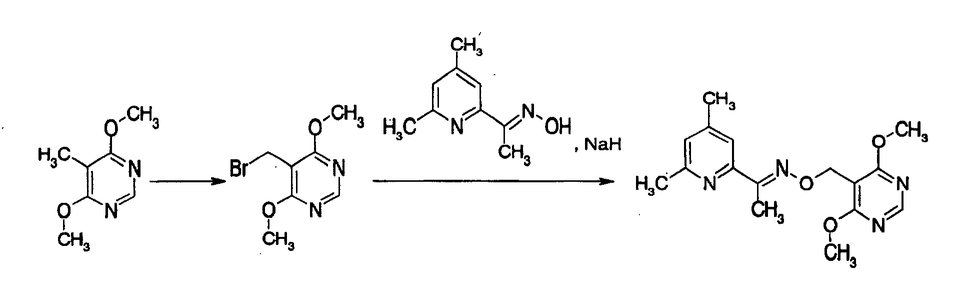

0.50 g (3.05 mmol) of 4,6-dimethoxy-5-methylpyridine was dissolved in 15 ml of carbon tetrachloride, and 0.58 g (3.26 mmol) of N-bromosuccinimide was added. The resulting solution was irradiated with light (infrared light 375 WR, produced by Toshiba Co., Ltd.) for 2 hours at the refluxing temperature. The reaction solution was cooled to room temperature. The deposited succinimide was separated by filtration. The filtrate was concentrated under reduced pressure to give a crude product of 5-bromomethyl-4,6-dimethoxypyridine.

Meanwhile, to a solution of 0.43 g (2.62 mmol) of 1-(4,6-dimethyl-2-pyridinyl)ethanone oxime in 10 ml of N,N-dimethylformamide was added 0.13 g (3.25 mmol) of sodium hydride (oiliness: 60%) while cooling in an ice bath, and the solution was stirred at the ice temperature for 30 minutes. To the resulting solution was added the whole amount of the previously prepared crude product of 5-bromomethyl-4,6-dimethoxypyridine while cooling in an ice bath, and the solution was stirred at room temperature for an hour. The reaction solution was poured into ice-water, and extracted with diethyl ether. The organic layer was washed with water, dried over anhydrous magnesium sulfate and concentrated under reduced pressure. The obtained crude product was purified by silica gel column chromatography [eluted with n-hexane:ethyl acetate = 7:3 (v/v)] to give 0.44 g of the target compound. Melting point: 125 to 126°C.

### Example 4

### Preparation of 4-cyano-6-methyl-2-pyridinecarboxyaldehyde O-[(2-methoxy-4-methyl-3-pyridinyl)methyl]oxime (Compound No. II-28)

### i) Preparation of 4-cyano-2,6-dimethylpyridine oxide

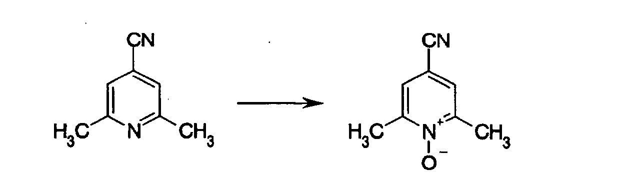

87.6 g (663 mmol) of 4-cyano-2,6-dimethylpyridine was dissolved in 200 ml of chloroform. To the solution was added 148 g (580 mmol) of m-chloroperbenzoic acid (70% equivalent) while cooling in an ice bath, and the solution was stirred over night at room temperature. The reaction mixture was diluted with chloroform. The organic layer was washed with an aqueous solution of sodium hydrogen carbonate, water and saturated salt water in this order, dried over anhydrous magnesium sulfate, and concentrated under reduced pressure. The obtained crude product was purified by silica gel column chromatography [eluted with chloroform:methanol = 20:1 (v/v)] to give 19 g of the target compound.

### ii) Preparation of 4-cyano-6-methyl-2-pyridinylmethanol

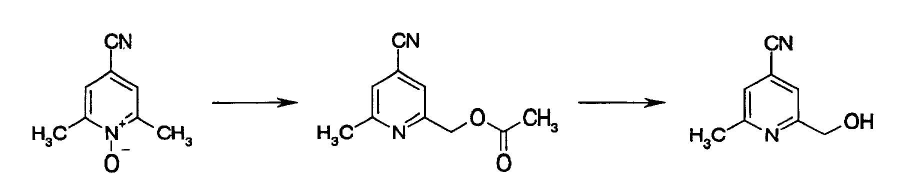

18.5 g (0.12 mol) of 4-cyano-2,6-dimethylpyridine-1-oxide was dissolved in 200 ml of acetic acid, followed by adding 13.5 g (0.132 mol) of acetic anhydride. The resulting solution was gradually raised to the refluxing temperature and stirred at the same temperature for 3 hours. The reaction solution was poured into water, neutralized with sodium bicarbonate, and extracted with ethyl acetate. The organic layer was washed with water, dried over anhydrous magnesium sulfate, and concentrated under reduced pressure to give 19.0 g of a crude product of 4-cyano-6-methyl-2-pyridinylmethyl acetate.

18.4 g (89.2 mmol) of the crude ester product was dissolved in 110 ml of THF, and then 75 ml of 1N sulfuric acid was added. The resulting solution was raised to the refluxing temperature and stirred for 17 hours. Another 36 ml of 1N sulfuric acid was added and the solution was refluxed for 3 hours. The reaction solution was cooled to room temperature, neutralized with aqueous sodium bicarbonate and extracted with chloroform. The organic layer was washed with saturated salt water, dried over anhydrous magnesium sulfate, and concentrated under reduced pressure to give 14.0 g of the target compound.

### iii) Preparation of 4-cyano-6-methyl-2-pyridinecarboxyaldehyde

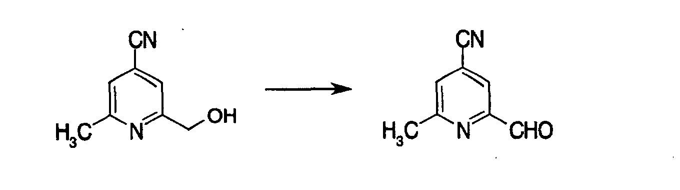

14 g of 4-cyano-6-methyl-2-pyridinylmethanol was dissolved in 200 ml of benzene. To the solution was added 22.2 g of activated manganese dioxide (product of Aldrich Co., Ltd.), and it was heated to the refluxing temperature, followed by stirring for 3 hours. The reaction mixture was cooled to room temperature. The insoluble matter was removed by filtration. The filtrate was concentrated under reduced pressure. The obtained crude crystals were washed with hexane to give 10 g of the target compound.

### iv) Preparation of 4-cyano-6-methyl-2-pyridinecarboxyaldehyde O-[(2-methoxy-4-methyl-3-pyridinyl)methyl]oxime

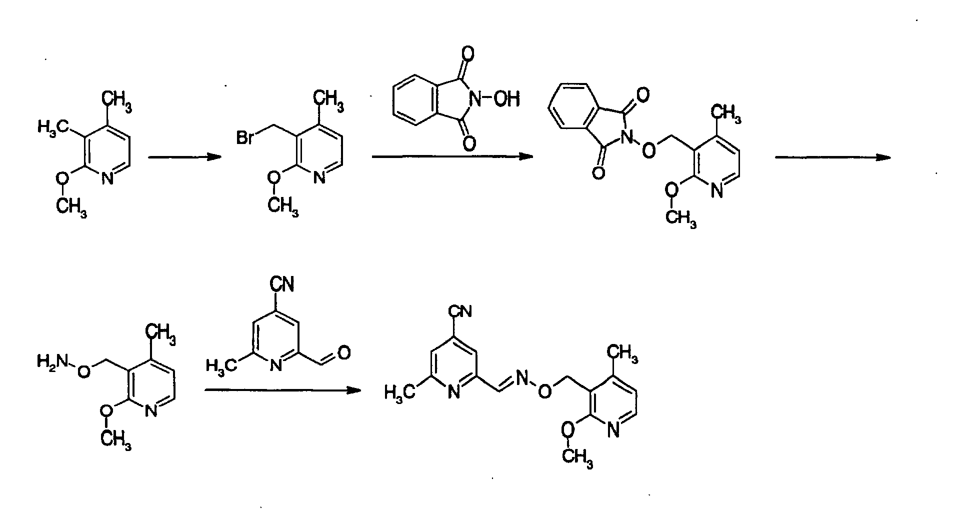

To a solution of 0.24 g (1.75 mmol) of 3,4-dimethyl-2-methoxypyridine in 10 ml of carbon tetrachloride was added 0.34 g (1.91 mmol) of N-bromosuccinimide. The resulting solution was irradiated with light (infrared light 375 WR, produced by Toshiba Co., Ltd.) for an hour at the refluxing temperature. The reaction solution was cooled to room temperature. The deposited succinimide was separated by filtration. The filtrate was concentrated under reduced pressure to give a crude product of 3-bromomethyl-2-methoxy-4-methylpyridine.

Meanwhile, to a solution of 0.29 g (1.78 mmol) of N-hydroxysuccinimide in 10 ml of N,N-dimethylformamide was added 0.19 g (1.88 mmol) of triethylamine. The resulting solution was heated to 70°C, and the whole amount of the previously prepared crude product of 3-bromomethyl-2-methoxy-4-methylpyridine was added therein, followed by stirring at 70°C for 2 hours. The reaction solution was cooled to room temperature, poured into ice-water, and extracted with ethyl acetate. The organic layer was washed with water, dried over anhydrous magnesium sulfate and concentrated under reduced pressure to give 0.38 g of a crude product of N-[(2-methoxy-4-methyl-3-pyridinyl)methyloxy]phthalimide.

The whole amount of the obtained crude product of N-[(2-methoxy-4-methyl-3-pyridinyl)methyloxy]phthalimide was dissolved in 10 ml of methanol, and 0.07 g (1.40 mmol) of hydrazine monohydrate was added therein, followed by stirring at room temperature for an hour. The reaction solution was concentrated under reduced pressure, and dissolved in ethyl acetate. The organic layer was washed with water, dried over anhydrous magnesium sulfate and concentrated under reduced pressure to give 0.15 g of a crude product of (2-methoxy-4-methyl-3-pyridinyl)methyloxyamine.

0.12 g (0.82 mmol) of 4-cyano-6-methyl-2-pyridinecarboxyaldehyde was dissolved in 10 ml of glacial acetic acid. To the resulting solution were added, at room temperature, 0.07 g (0.85 mmol) of sodium acetate and then the whole amount of the previously prepared (2-methoxy-4-methyl-3-gyridinyl)methyloxyamine, and the solution was stirred further at room temperature for 2 hours. The reaction solution was poured into ice-water, and extracted with ethyl acetate. The ethyl-acetate layer was neutralized with a 5% aqueous solution of sodium hydrogen carbonate, washed with saturated salt water, dried over anhydrous magnesium sulfate and concentrated under reduced pressure. The obtained crude product was purified by silica gel column chromatography [eluted with n-hexane:ethyl acetate = 7:3 (v/v)] to give 0.18 g of the target compound. Melting point: 106 to 108°C.

### Example 5

### Preparation of 4-cyano-6-methyl-2-pyridinecarboxyaldehyde 0-[(2-,4-dimethoxy-3-pyridinyl)methyl]oxime (Compound No. II-27)

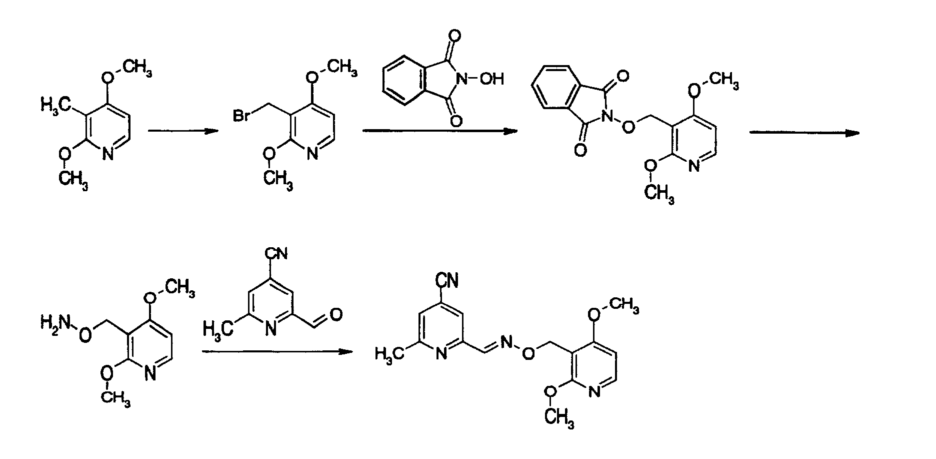

To a solution of 0.30 g (1.96 mmol) of 2,4-dimethoxy-3-methylpyridine in 10 ml of carbon tetrachloride was added 0.38 g (2.13 mmol) of N-bromosuccinimide. The resulting solution was irradiated with light (infrared light 375 WR, produced by Toshiba Co., Ltd.) for an hour at the refluxing temperature. The reaction solution was cooled to room temperature. The deposited succinimide was separated by filtration. The filtrate was concentrated under reduced pressure to give a crude product of 3-bromomethyl-2,4-dimethoxypyridine.

To a solution of 0.32 g (1.96 mmol) of N-hydroxyphthalimide in 10 ml of N,N-dimethylformamide was added 0.22 g (2.18 mmol) of triethylamine. The resulting solution was heated to 70°C, and the whole amount of the previously prepared crude product of 3-bromomethyl-2,4-dimethoxypyridine was added therein, followed by stirring at 70°C for 2 hours. The reaction solution was cooled to room temperature, poured into ice-water, and extracted with ethyl acetate. The organic layer was washed with water, dried over anhydrous magnesium sulfate and concentrated under reduced pressure to give 0.57 g of a crude product of N-[(2,4-dimethoxy-3-pyridinyl)methyloxy]phthalimide.

The whole amount of the obtained crude product of N-[(2,4-dimethoxy-3-pyridinyl)methyloxy]phthalimide was dissolved in 10 ml of methanol, and 0.1 g (2.0 mmol) of hydrazine monohydrate was added therein, followed by stirring at room temperature for an hour. The reaction solution was concentrated under reduced pressure, and dissolved in ethyl acetate. The organic layer was washed with water, dried over anhydrous magnesium sulfate and concentrated under reduced pressure to give 0.34 g of a crude product of (2,4-dimethoxy-3-pyridinyl)methyloxyamine.

0.24 g (1.65 mmol) of 4-cyano-6-methyl-2-pyridinecarboxyaldehyde was dissolved in 10 ml of glacial acetic acid. To the resulting solution were added, at room temperature, 0.14 g (1.70 mmol) of sodium acetate and then the whole amount of the previously prepared (2,4-dimethoxy-3-pyridinyl)methyloxyamine, and the solution was stirred further at room temperature for 2 hours. The reaction solution was poured into ice-water, and extracted with ethyl acetate. The ethyl acetate layer was neutralized with a 5% aqueous solution of sodium hydrogen carbonate, washed with saturated salt water, dried over anhydrous magnesium sulfate and concentrated under reduced pressure. The obtained crude product was purified by silica gel column chromatography [eluted with benzene:ethyl acetate = 9:1 (v/v)] to give 0.14 g of the target compound. Melting point: 130 to 132°C.

### Example 6

### Preparation of 4-cyano-6-methyl-2-pyridinecarboxyaldehyde O-[(4,6-dimethoxy-5-pyrimidinyl)methyl]oxime (Compound No. II-29)

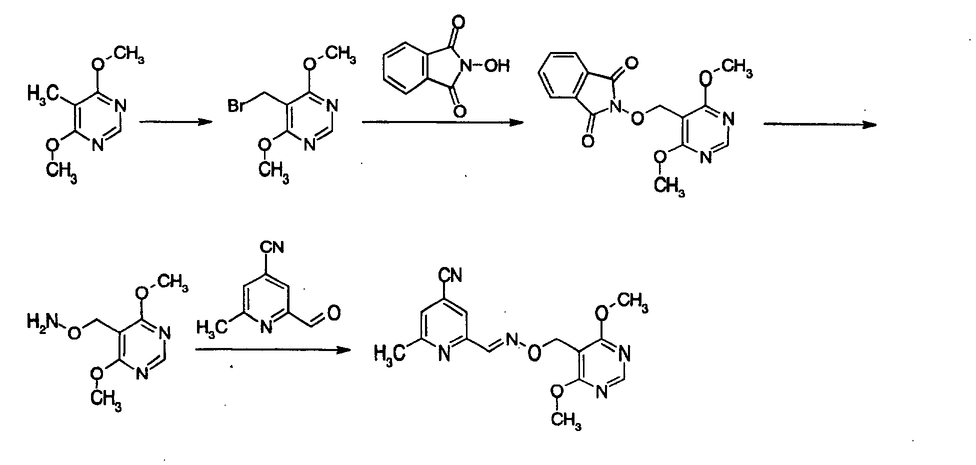

To a solution of 1.0 g (6.49 mmol) of 4,6-dimethoxy-5-methylpyrimidine in 10 ml of carbon tetrachloride was added 1.27 g (7.13 mmol) of N-bromosuccinimide. . The resulting solution was irradiated with light (infrared light 375 WR, produced by Toshiba Co., Ltd.) for an hour at the refluxing temperature. The reaction solution was cooled to room temperature. The deposited succinimide was separated by filtration. The filtrate was concentrated under reduced pressure to give a crude product of 5-bromomethyl-4,6-dimethoxypyrimidine.

Meanwhile, to a solution of 1.06 g (6.50 mmol) of N-hydroxyphthalimide in 10 ml of N,N-dimethylformamide was added 0.72 g (7.13 mmol) of triethylamine. The resulting solution was heated to 70°C, and the whole amount of the previously prepared crude product of 5-bromomethyl-4,6-dimethoxypyrimidine was added therein, followed by stirring at 70°C for 2 hours. The reaction solution was cooled to room temperature, poured into ice-water, and extracted with ethyl acetate. The organic layer was washed with water, dried over anhydrous magnesium sulfate and concentrated under reduced pressure to give 1.46 g of a crude product of N-[(4,6-dimethoxy-5-pyrimidinyl)methyloxy]phthalimide.

The whole amount of the obtained crude product of N-[(4,6-dimethoxy-5-pyrimidinyl)methyloxy]phthalimide was dissolved in 10 ml of methanol, and 0.28 g (5.60 mmol) of hydrazine monohydrate was added therein, followed by stirring at room temperature for an hour. The reaction solution was concentrated under reduced pressure, and dissolved in ethyl acetate. The organic layer was washed with water, dried over anhydrous magnesium sulfate and concentrated under reduced pressure to give 0.67 g of a crude product of (4,6-dimethoxy-5-pyrimidinyl)methyloxyamine.

0.67 g (4.59 mmol) of 4-cyano-6-methyl-2-pyridinecarboxyaldehyde was dissolved in 10 ml of glacial acetic acid. To the resulting solution were added, at room temperature, 0.37 g (4.51 mmol) of sodium acetate and then the whole amount of the previously prepared (4,6-dimethoxy-5-pyrimidinyl)methyloxyamine, and the solution was stirred further at room temperature for 2 hours. The reaction solution was poured into ice-water, and extracted with ethyl acetate. The ethyl-acetate layer was neutralized with a 5% aqueous solution of sodium hydrogen carbonate, washed with saturated salt water, dried over anhydrous magnesium sulfate and concentrated under reduced pressure. The obtained crude product was purified by silica gel column chromatography [eluted with benzene:ethyl acetate = 9:1 (v/v)] to give 0.43 g of the target compound. Melting point: 160 to 161°C.

### Example 7

### Preparation of 4-cyano-6-methyl-2-pyridinecarboxyaldehyde O-[(2-fluoro-4-methoxy-3-pyridinyl)methyl]oxime (Compound No. II-75)

### i) Preparation of 2-fluoro-3-hydroxymethyl-4-methoxypyridine

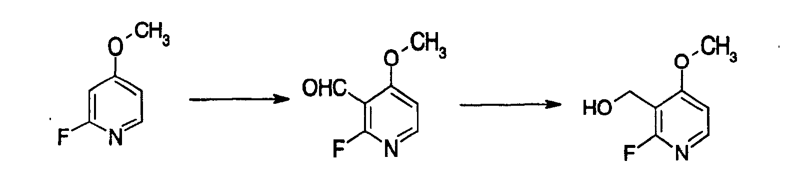

To 10 ml of well-dehydrated THF was added 0.4 g (3.1 mmol) of 2-fluoro-6-methoxy-3-pyridine, and 1.23 ml (3.2 mmol) of n-butyl lithium (a 2.6M hexane solution) was dropped at -78°C under nitrogen atmosphere, followed by stirring for an hour. To the solution was added 0.73 g (10 mmol) of N,N-dimethylformamide at the same temperature and the solution was stirred for another hour. The solution was gradually warmed to 0°C and 10 ml of a 10% solution of ammonium chloride was added. The obtained reaction mixture was extracted with diethyl ether. The organic layer was washed with saturated salt water, dried over anhydrous magnesium sulfate and concentrated under reduced pressure to give 0.4 g of crude 2-fluoro-6-methoxypyridinecarboxyaldehyde.

0.4 g of the obtained crude 2-fluoro-6-methoxypyridinecarboxyaldehyde was dissolved in 6 ml of THF. To the solution was added 0.06 g (1.55 mmol) of NaBH₄ while cooling in an ice bath, and the solution was stirred at room temperature for 3 hours. The reaction solution was poured into water, neutralized with 3N hydrochloric acid and extracted with ethyl acetate. The organic layer was washed with water, dried over anhydrous magnesium sulfate and concentrated under reduced pressure to give 0.3 g of the target compound.

### ii) Preparation of 4-cyano-6-methyl-2-pyridinecarboxyaldehyde O-[(2-fluoro-4-methoxy-3-pyridinyl)methyl]oxime

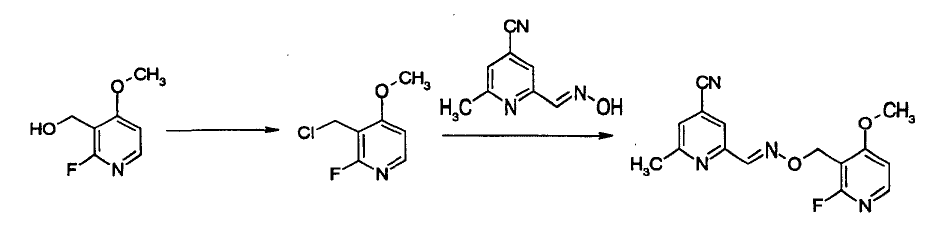

0.13 g (0.83 mmol) of 2-fluoro-3-hydroxymethyl-4-methoxypyridine was dissolved in 5 ml of benzene and cooled to 5°C. To this solution was added 0.1 g (0.84 mmol) of thionyl chloride at the same temperature. The solution was warmed to room temperature and stirred for 60 minutes. The reaction solution was washed with saturated aqueous sodium bicarbonate, dried over anhydrous magnesium sulfate and concentrated under reduced pressure to give a crude product of 3-chloromethyl-2-fluoro-4-methoxypyridine.

Meanwhile, 0.16 g (1.0 mmol) of 4-cyano-6-methyl-2-pyridinecarboxyaldoxime was dissolved in 5 ml of N,N-dimethylformamide. To the solution was added 0.04 g (1.0 mmol) of sodium hydride (oiliness: 60%) while cooling in an ice bath, and the solution was stirred at the ice temperature for 15 minutes. To the resulting solution was added the whole amount of the previously prepared crude product of 3-chloromethyl-2-fluoro-4-methoxypyridine while cooling in an ice bath, and the solution was stirred at room temperature for 2 hours. The reaction mixture was poured into ice-water, and extracted with diethyl ether. The organic layer was washed with saturated salt water, dried over anhydrous magnesium sulfate and concentrated under reduced pressure. The obtained crude product was purified by silica gel column chromatography [eluted with hexane:ethyl acetate = 1:1 (v/v)] to give 0.08 g of the target compound. Melting point: 150 to 152°C.

### Example 8

Preparation of 4-chloro-6-methyl-2-pyridinecarboxyaldehyde O-[(4,6-dimethoxy-5-pyrimidinyl)methyl]oxime (Compound No. II-20)

### i) Preparation of 4-chloro-6-methyl-2-pyridinylmethanol

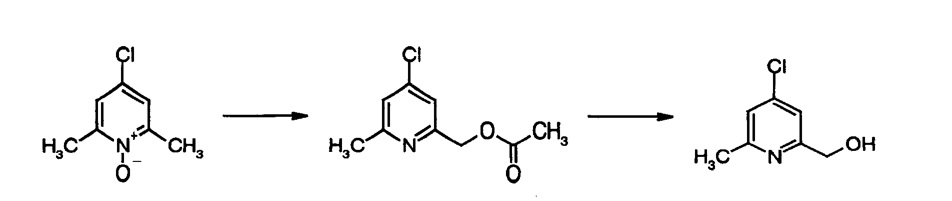

To 40 ml of acetic anhydride heated to 70°C was added, little by little, 19.8 g (125.6 mmol) of 4-chloro-2,6-dimethylpyridine-1-oxide. The solution was gradually heated to the refluxing temperature and stirred at the same temperature for 4 hours. The reaction solution was concentrated under reduced pressure, and water was added, followed by extracting with chloroform. The organic layer was washed with saturated aqueous sodium bicarbonate, dried over anhydrous magnesium sulfate and concentrated under reduced pressure to give 24.2 g of a crude product of 4-chloro-6-methyl-2-pyridinylmethyl acetate.

To 150 ml of methanol was added 3.0 g of potassium hydroxide, and a solution of the crude ester product in 10 ml of methanol was dropped at room temperature, and the solution was stirred at room temperature for 4 hours. The reaction solution was concentrated under reduced pressure. The obtained reaction mixture was diluted with water, neutralized with dilute hydrochloric acid, and extracted with ethyl acetate. The organic layer was washed with saturated salt water, dried over anhydrous magnesium sulfate and concentrated under reduced pressure to give 14.0 g of the target compound.

### ii) Preparation of 4-chloro-6-methyl-2-pyridinecarboxyaldehyde

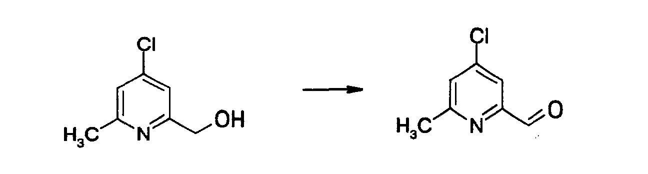

14.0 g (88.8 mmol) of 4-chloro-6-methyl-2-pyridinylmethanol was dissolved in 100 ml of benzene, and 28.0 g of activated manganese dioxide was added. The resulting solution was refluxed for 3 hours. The reaction solution was cooled. The insoluble matter was separated by filtration through celite. The filtrate was concentrated under reduced pressure to give 6.1 g of the target compound.

### iii) Preparation of 4-chloro-6-methyl-2-pyridinecarboxyaldehyde O-[(4,6-dimethoxy-5-pyrimidinyl)methyl]oxime

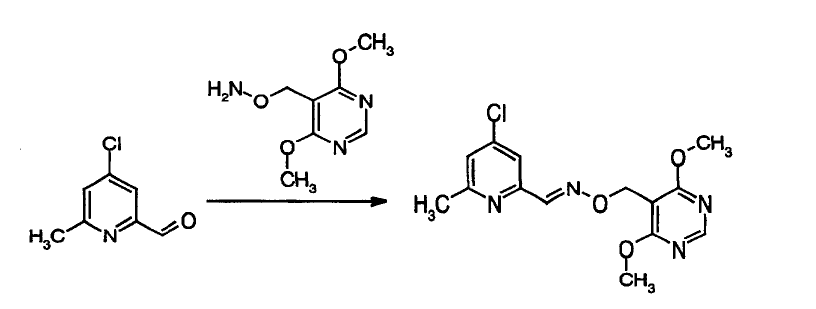

0.17 g (1.1 mmol) of 4-chloro-6-methyl-2-pyridinecarboxyaldehyde was dissolved in 5 ml of acetic acid and 0.09 g (1.1 mmol) of sodium acetate was added at room temperature. Further, 0.2 g (1.1 mmol) of (4,6-dimethoxy-5-pyrimidinyl)methyloxyamine was added at the same temperature followed by stirring for 2 hours. The reaction mixture was concentrated under reduced pressure, neutralized with an aqueous solution of sodium hydrogen carbonate, and extracted with ethyl acetate. The organic layer was washed with saturated salt water, dried over anhydrous magnesium sulfate and concentrated under reduced pressure. The obtained crude product was purified by silica gel column chromatography [eluted with hexane:ethyl acetate = 7:3 (v/v)] to give 0.18 g of the target compound. Melting point: 135 to 137°C.

### Example 9

### Preparation of 6-methyl-4-trifluoromethyl-2-pyridinecarboxyaldehyde O-[(4,6-dimethoxy-5-pyrimidinyl)methyl]oxime (Compound No. II-36)

### i) Preparation of ethyl 6-methyl-4-trifluoromethyl-2-pyridinecarboxylate

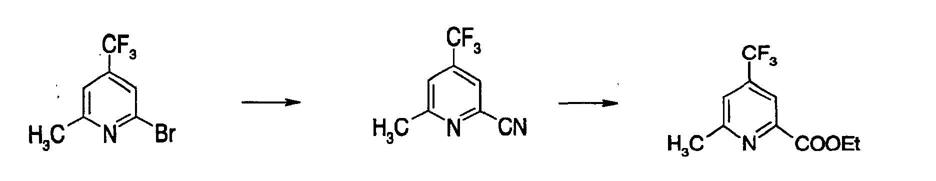

11.1 g (46.3 mmol) of 2-bromo-6-methyl-4-trifluoromethylpyridine was dissolved in 90 ml of DMF and 4.55 g (50.9 mmol) of copper cyanide and 7.63 g (50.9 mmol) of sodium iodide were added. The resulting solution was refluxed for 6 hours. The reaction solution was cooled to room temperature, poured into cold water, and extracted with ethyl acetate. The organic layer was washed with water and saturated salt water, dried over magnesium sulfate and concentrated under reduced pressure. The obtained crude product was purified by silica gel column chromatography [eluted with hexane:ethyl acetate = 7:3 (v/v)] to give 7.44 g of 2-cyano-6-methyl-4-trifluoromethylpyridine.

4.0 g (21.5 mmol) of the obtained 2-cyano-6-methyl-4-trifluoromethylpyridine was dissolved in 7 ml of ethanol, and 7 ml of concentrated sulfuric acid was added with stirring at room temperature. The resulting solution was refluxed for 2 hours. The reaction solution was cooled to room temperature, poured into cold water, neutralized with sodium carbonate, and extracted with ethyl acetate. The organic layer was washed with saturated salt water, dried over magnesium sulfate and concentrated under reduced pressure. The obtained crude product was purified by silica gel column chromatography [eluted with hexane:ethyl acetate = 7:3 (v/v)] to give 3.2 g of the target compound.

### ii) Preparation of 2-hydroxymethyl-6-methyl-4-trifluoromethylpyridine

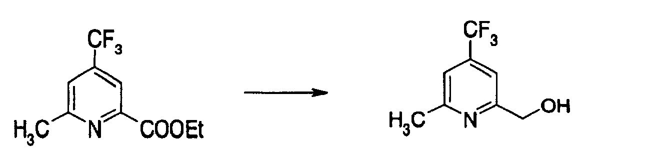

LiAlH₄ was added to 10 ml of dehydrated THF, and a solution of 3.2 g (13.7 mmol) of ethyl 6-methyl-4-trifluoromethyl-2-pyridinecarboxylate in 27 ml of THF was dropped while cooling in an ice bath. The resulting solution was warmed to room temperature and stirred for an hour. 40 ml of diethyl ether was added to the reaction solution, and 3.0 ml of ethyl acetate was dropped therein, followed by stirring for 30 minutes. Water was added to the reaction solution to neutralize with 1N hydrochloric acid. The insoluble matter was separated by filtration through celite. The filtrate was extracted with ethyl acetate. The organic layer was washed with saturated salt water, dried over magnesium sulfate and concentrated under reduced pressure. The obtained crude product was purified by silica gel column chromatography [eluted with hexane:ethyl acetate = 7:3 (v/v)] to give 0.58 g of the target compound.

### iii) Preparation of 6-methyl-4-trifluoromethyl-2-pyridinecarboxyaldoxime

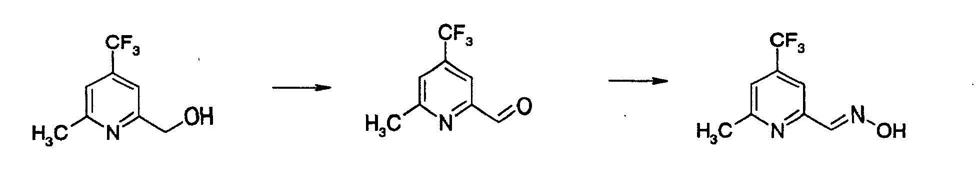

1.04 g (5.44 mmol) of 2-hydroxymethyl-6-methyl-4-trifluoromethylpyridine was dissolved in 11 ml of benzene, and 2.0 g of activated manganese dioxide was added therein. The resulting mixture was refluxed for 20 hours. The reaction mixture was cooled. The insoluble matter was separated by filtration through celite. The filtrate was concentrated under reduced pressure to give 1.03 g of 6-methyl-4-trifluoromethyl-2-pyridinecarboxyaldehyde.

The whole amount of the obtained crude 6-methyl-4-trifluoromethyl-2-pyridinecarboxyaldehyde was dissolved in 12 ml of methanol, and 0.46 g (6.7 mmol) of hydroxylamine hydrochloride was added therein. The resulting solution was refluxed for 2.5 hours. The reaction solution was cooled and concentrated under reduced pressure. Water was added to the solution, followed by neutralizing with a 1N solution of sodium hydroxide, and extracted with ethyl acetate. The organic layer was washed with saturated salt water, dried over magnesium sulfate and concentrated under reduced pressure. The obtained crude product was purified by silica gel column chromatography [eluted with hexane:ethyl acetate = 7:3 (v/v)] to give 1.05 g of the target compound.

### iv) Preparation of 6-methyl-4-trifluoromethyl-2-pyridinecarboxyaldoxime O-[(4,6-dimethoxy-3-pyrimidinyl)methyl]ether

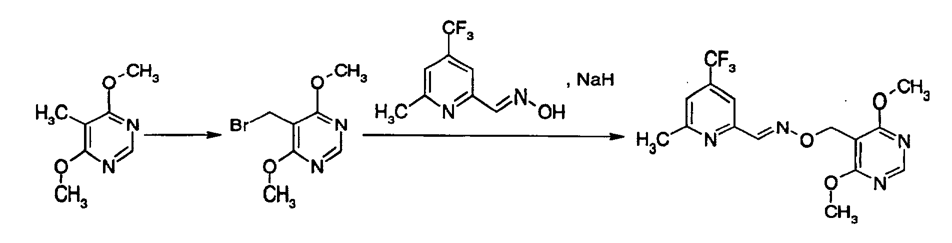

0.207 g (1.34 mmol) of 4,6-dimethoxy-3-methylpyrimidine was dissolved in 5 ml of carbon tetrachloride, and 0.26 g (1.48 mmol) of N-bromosuccinimide was added therein. The resulting solution was irradiated with light (infrared light 375 WR, produced by Toshiba Co., Ltd.) for 2 hours at the refluxing temperature. The reaction solution was cooled to room temperature. The deposited succinimide was separated by filtration. The filtrate was concentrated under reduced pressure to give a crude product of 3-bromomethyl-4,6-dimethoxypyridine.

Meanwhile, 0.25 g (1.23 mmol) of 6-methyl-4-trifluoromethyl-2-pyridinecarboxyaldoxime was dissolved in 5 ml of N,N-dimethylformamide. To the solution was added 73 mg (1.84 mmol) of sodium hydride (oiliness: 60%) while cooling in an ice bath, and the solution was stirred at the ice temperature for 30 minutes. To the resulting solution was added the whole amount of the previously prepared crude product of 3-bromomethyl-4,6-dimethoxypyridine while cooling in an ice bath, and the solution was stirred at room temperature for 40 minutes. The reaction mixture was poured into ice-water, and extracted with ethyl acetate. The organic layer was washed with water, dried over anhydrous magnesium sulfate and concentrated under reduced pressure. The obtained crude product was purified by silica gel column chromatography [eluted with hexane:ethyl acetate = 10:1 (v/v)] to give 0.11 g of the target compound. Melting point: 121 to 123°C.

### Example 10

Preparation of 4,6-dimethyl-2-pyridinecarboxyaldehyde O-[(5-chloro-1,3-dimethylpyrazol-4-yl)methyloxime (Compound No. II-8)

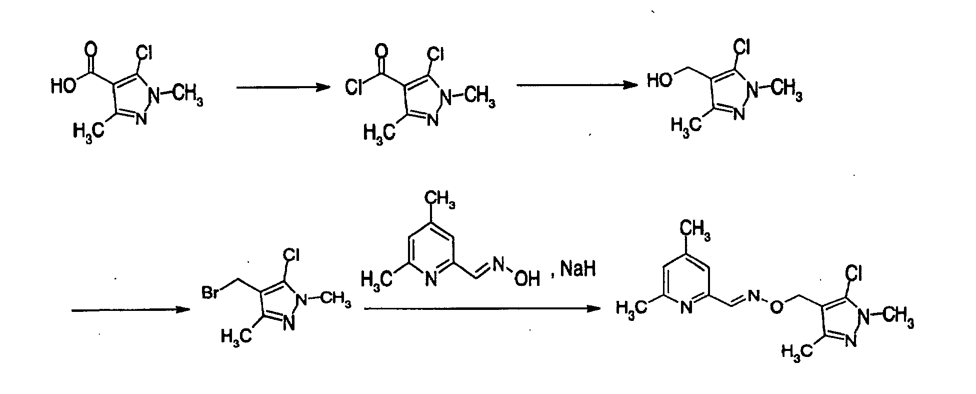

To a solution of 1.7 g (10.0 mmol) of 5-chloro-1,3-dimethylpyrazole-4-carboxylic acid in 10 ml of chloroform was added 1.43 g (12.0 mmol) of thionyl chloride. The resulting solution was refluxed for 2 hours. The reaction solution was cooled to room temperature and concentrated under reduced pressure. 20 ml of THF and then a solution of 1.89 g of sodium borohydride in 5 ml of water were added to the solution, and the solution was stirred at room temperature for 2 hours. To the solution was added 10 ml of 2N hydrochloric acid, followed by stirring at room temperature for 30 minutes, and extracted with ethyl acetate. The organic layer was neutralized with a 5% aqueous solution of sodium hydrogen carbonate, washed with saturated salt water, dried over anhydrous magnesium sulfate and concentrated under reduced pressure. The obtained crude product was purified by silica gel column chromatography [eluted with chloroform:ethyl acetate = 7:3 (v/v)] to give 1.6 g of 5-chloro-1,3-dimethyl-4-hydroxymethylpyrazole.

To a solution of 0.8 g (5.00 mmol) of 5-chloro-1,3-dimethyl-4-hydroxymethylpyrazole in 10 ml of dichloromethane were added 1.7 g (6.50 mmol) of triphenylphosphine and then 2.5 g (7.53 mmol) of carbon tetrabromide. The resulting solution was stirred at room temperature for 2 hours. Dichloromethane was distilled off under reduced pressure by a rotary evaporator to give crude 4-bromomethyl-5-chloro-1,3-dimethylpyrazole.

Meanwhile, to a solution of 0.75 g (5.00 mmol) of 4,6-dimethyl-2-pyridinecarboxyaldehyde oxime in 10 ml of N,N-dimethylformamide was added 0.20 g (5.00 mmol) of sodium hydride (oiliness: 60%) while cooling in an ice bath, and the solution was stirred at the ice temperature for 30 minutes. To the resulting solution was added the whole amount of the previously prepared crude 4-bromomethyl-5-chloro-1,3-dimethylpyrazole while cooling in an ice bath. The reaction solution was stirred for 2 hours while cooling in an ice bath, then poured into ice-water, and extracted with ethyl acetate. The ethyl-acetate layer was washed with water, dried over anhydrous magnesium sulfate and concentrated under reduced pressure. The obtained crude product was purified by silica gel column chromatography [eluted with chloroform: ethyl acetate = 7:3 (v/v)] to give 0.7 g of the target compound. Melting point: 77 to 78°C.

### Example 11

Preparation of 4-cyano-6-methylpyridinyl-2-carboxyaldehyde [(4-methoxy-3-thienyl)methyl]ether (Compound No. II-77)

### i) Preparation of 3-hydroxymethyl-4-methoxythiophene

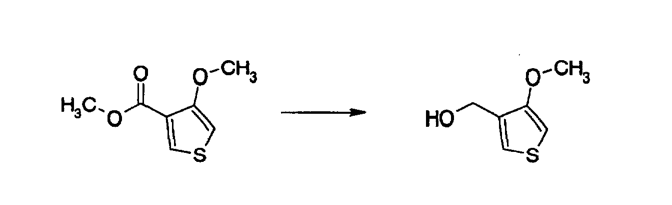

0.13 g (3.5 mmol) of lithium aluminum hydride was added to 6 ml of well-dehydrated THF while cooling at -20°C, and a solution of methyl 4-methoxy-3-thiophenecarboxylate in 10 ml of well dehydrated THF was dropped over 15 minutes at the same temperature. The resulting solution was stirred at the same temperature for 30 minutes, and 1 ml of water and then 1 ml of a 4N aqueous solution of sodium hydroxide were added therein, followed by stirring at room temperature for 15 minutes. Further 1 ml of water was added followed by stirring for 80 minutes. The insoluble matter was removed by filtration. The filtrate was concentrated under reduced pressure to remove THF. The obtained aqueous solution was extracted with ethyl acetate. The organic layer was dried over magnesium sulfate and concentrated under reduced pressure to give 0.4 g of the target compound.

### ii) Preparation of (4-methoxy-3-thienyl)methyloxyamine

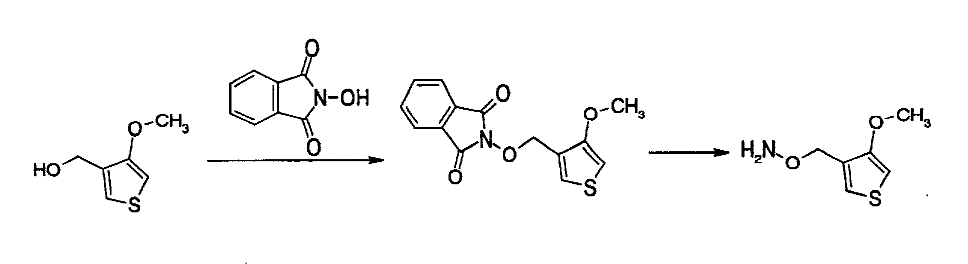

The whole amount of the previously prepared 3-hydroxymethyl-4-methoxythiophene was dissolved in 25 ml of THF, and 0.48 g (2:94 mmol) of N-hydroxysuccinimide and 0.77 g (2.94 mmol) of triphenylphosphine were added therein. The resulting solution was cooled to -40°C, and 1.33 ml of a 40% toluene solution of DEAD was dropped in 5 minutes. The solution was gradually warmed to room temperature and stirred overnight. The reaction solution was concentrated under reduced pressure. The obtained reaction mixture was purified by silica gel column chromatography [eluted with benzene:ethyl acetate = 30:1 (v/v)] to give 0.65 g of N-[(4-methoxy-3-thienyl)methyloxy]succinimide.

0.65 g (2.2 mmol) of N-[(4-methoxy-3-thienyl)methyloxy]succinimide was suspended in 20 ml of methanol, and 0.16 g (3.2 mmol) of hydrazine monohydrate was added at room temperature followed by stirring at room temperature for 4 hours. The reaction solution was concentrated under reduced pressure and dissolved in diethyl ether. The insoluble matter was removed by filtration. The filtrate was washed with water, dried over anhydrous magnesium sulfate and concentrated under reduced pressure to give 0.40 g of a crude product of (2-methoxy-4-methyl-3-pyridyl)methyloxyamine.

### iii) Preparation of 4-cyano-6-methylpyridinyl-2-carboxyaldehyde [(4-methoxy-3-thienyl)methyl]ether

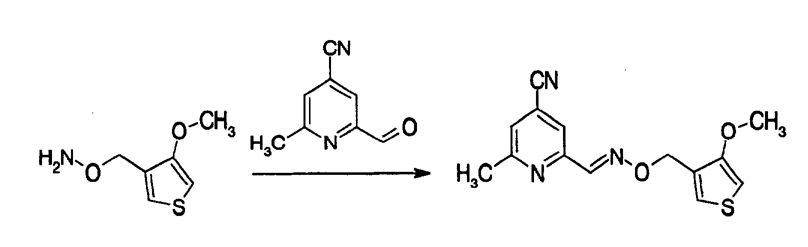

0.10 g (0.63 mmol) of 4-cyano-6-methyl-2-pyridinecarboxyaldehyde was dissolved in 10 ml of glacial acetic acid, and 0.1 g of the previously prepared crude product of (2-methoxy-4-methyl-3-pyridyl)methyloxyamine was added therein. The resulting solution was stirred for 4 hours at room temperature. The reaction solution was poured into ice-water, and extracted with ethyl acetate. The ethyl-acetate layer was neutralized with a 5% aqueous solution of sodium hydrogen carbonate, washed with saturated salt water, dried over anhydrous magnesium sulfate and concentrated under reduced pressure. The obtained crude product was purified by silica gel column chromatography [eluted with hexane:ethyl acetate = 10:1 (v/v)] to give 0.15 g of the target compound. Melting point: 96 to 98°C.

### Example 12

Preparation of 6-methylpyridinyl-2-carboxyaldehyde 3-furylmethylether (Compound No. II-70)

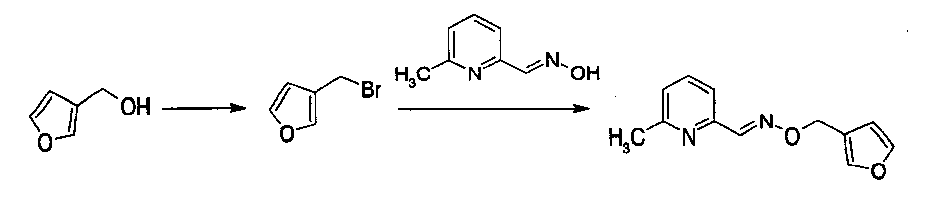

To a solution of 0.98 g (10.0 mmol) of 3-hydroxymethylfuran in 20 ml of dichloromethane were added 3.4 g (13.0 mmol) of triphenylphosphine and then 5.0 g (15.0 mmol) of carbon tetrabromide. The resulting solution was stirred at room temperature for 2 hours. Dichloromethane was distilled off under reduced pressure by a rotary evaporator to give crude 3-bromomethylfuran.

Meanwhile, 1.21 g (10.0 mmol) of 6-dimethyl-2-pyridinecarboxyaldoxime was dissolved in 10 ml of N,N-dimethylformamide and the whole amount of the previously prepared crude 3-bromomethylfuran was added therein. While this solution was being stirred at room temperature, 1.2 g (10.07 mmol) of a 50% aqueous solution of potassium hydroxide was dropped, and further stirred at room temperature for 2 hours. The reaction solution was poured into ice-water, and extracted with ethyl acetate. The ethyl acetate layer was washed with water, dried over anhydrous magnesium sulfate and concentrated under reduced pressure. The obtained crude product was purified by silica gel column chromatography [eluted with chloroform:ethyl acetate = 7:3 (v/v)] to give 0.2 g of the target compound. Refractive index: n_{D} 20.6-1.5535.

### Example 13

### Preparation of N-(2,4-dimethoxy-3-pyridinyl)methyloxy-4-chloro-6-methyl-2-pyridinecarboxyimide amide (Compound No. II-23)

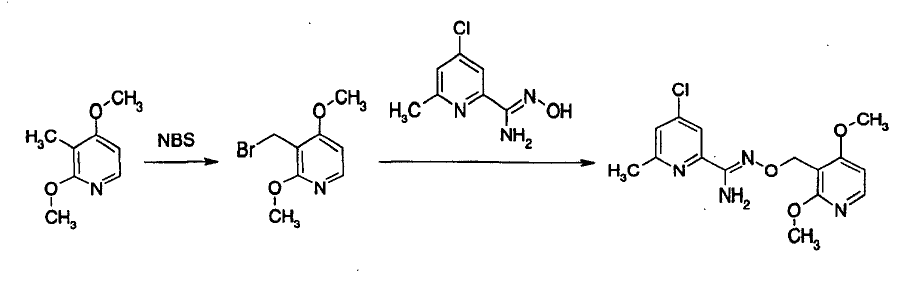

To a solution of 0.23 g (1.50 mmol) of 2,4-dimethoxy-3-methylpyridine in 5 ml of carbon tetrachloride was added 0.29 g (1.63 mmol) of N-bromosuccinimide. . The resulting solution was irradiated with light (infrared light 375 WR, produced by Toshiba Co., Ltd.) for an hour at the refluxing temperature. The solution was cooled to room temperature. The deposited succinimide was separated by filtration. The filtrate was concentrated under reduced pressure to give a crude product of 3-bromomethyl-2,4-dimethoxypyridine.

Meanwhile, to a solution of 0.25 g (1.34 mmol) of N-hydroxy-4-chloro-6-methyl-2-pyridinecarboxyimide amide in 5 ml of N,N-dimethylformamide was added 0.06 g (1.50 mmol) of sodium hydride (oiliness: 60%) while cooling in an ice bath, and the solution was stirred at the ice temperature for 30 minutes. To the resulting solution was added the whole amount of the previously prepared crude product of 3-bromomethyl-2,4-dimethoxypyridine while cooling in an ice bath, and the solution was stirred at room temperature for 2 hours. The reaction mixture was poured into ice-water, and extracted with diethyl ether. The organic layer was washed with water, dried over anhydrous magnesium sulfate and concentrated under reduced pressure. The obtained crude product was purified by silica gel column chromatography [eluted with n-hexane:ethyl acetate = 7:3 (v/v)] to give 0.22 g of the target compound. Melting point: 149 to 150°C.

### Example 14

### Preparation of 2-(4,6-dimethyl-2-pyridinyl)-2-[(2,4-dimethoxy-3-pyrimidinyl)methyloxyimino]acetonitrile (Compound No. II-34)

### i) Preparation of 2-(4,6-dimethyl-2-pyridinyl)-2-hydroxyiminoacetonitrile

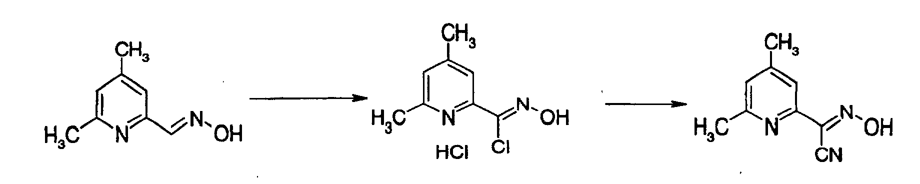

5.0 g (33.3 mmol) of 4,6-dimethyl-2-pyridinecarboxyaldoxime was suspended in 50 ml of chloroform. Chlorine gas was blown in for 20 minutes while the solution was being stirred at a temperature below 10°C, and stirred for 25 minutes at the same temperature. The deposited crystals were separated by filtration, and washed with diethyl ether to give 5.98 g of (4,6-dimethyl-2-pyridinyl) chloroformaldoxime hydrochloride.

1.5 g (8.09 mmol) of the obtained hydrochloride was added to 30 ml of chloroform. To the resulting solution were dropped, at -5°C, 1.79 g (17.8 mmol) of triethylamine and then a solution of 0.48 g (9.7 mmol) of sodium cyanide in 28 ml of water. The resulting solution was gradually warmed to room temperature, and stirred at room temperature for 6 hours. The reaction solution was separated. The organic layer was washed with saturated salt water, dried over anhydrous magnesium sulfate and concentrated under reduced pressure. The obtained crude product was purified by silica gel column chromatography [eluted with benzene:ethyl acetate = 4:1 (v/v)] to give 0.68 g of the target compound.

### ii) Preparation of 2-(4,6-dimethyl-2-pyridinyl)-2-[(2,4-dimethoxy-3-pyridinyl)methyloxyimino]acetonitrile

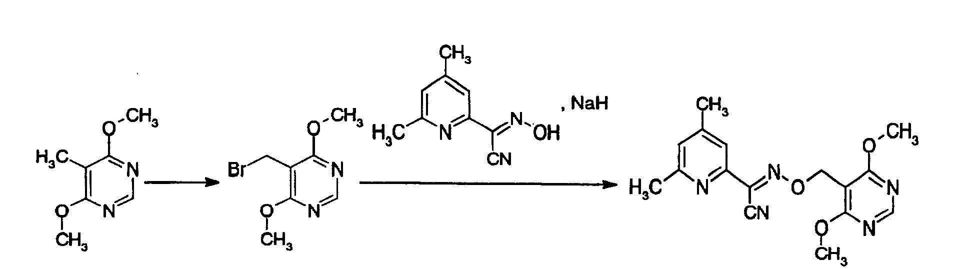

0.30 g (1.94 mmol) of 4,6-dimethoxy-5-methylpyrimidine was dissolved in 4 ml of carbon tetrachloride, and 0.38 g (2.13 mmol) of N-bromosuccinimide was added therein. The resulting solution was irradiated with light (infrared light 375 WR, produced by Toshiba Co., Ltd.) for 2 hours at the refluxing temperature. The solution was cooled to room temperature. The deposited succinimide was separated by filtration. The filtrate was concentrated under reduced pressure to give a crude product of 5-bromomethyl-4,6-dimethoxypyrimidine.

Meanwhile, to a solution of 0.34 g (1.94 mmol) of 2-(4,6-dimethyl-2-pyridinyl)-2-oxoacetonitrile in 5 ml of N,N-dimethylformamide was added 0.12 g (2.9 mmol) of sodium hydride (oiliness: 60%) while cooling in an ice bath, and the solution was stirred at the ice temperature for 30 minutes. To the resulting solution was added the whole amount of the previously prepared crude product of 5-bromomethyl-4,6-dimethoxypyrimidine while cooling in an ice bath, and the solution was stirred at room temperature for an hour. The reaction solution was poured into ice-water, and extracted with ethyl acetate. The organic layer was washed with water, dried over anhydrous magnesium sulfate and concentrated under reduced pressure. The obtained crude crystals were washed with a small amount of acetone to give 0.24 g of the target compound. Melting point: 196 to 201°C.

The compounds of the present invention that were produced according to the methods mentioned above are shown in Table 2. The abbreviations and symbols used in Table 2 have the same meanings as those for Table 1.

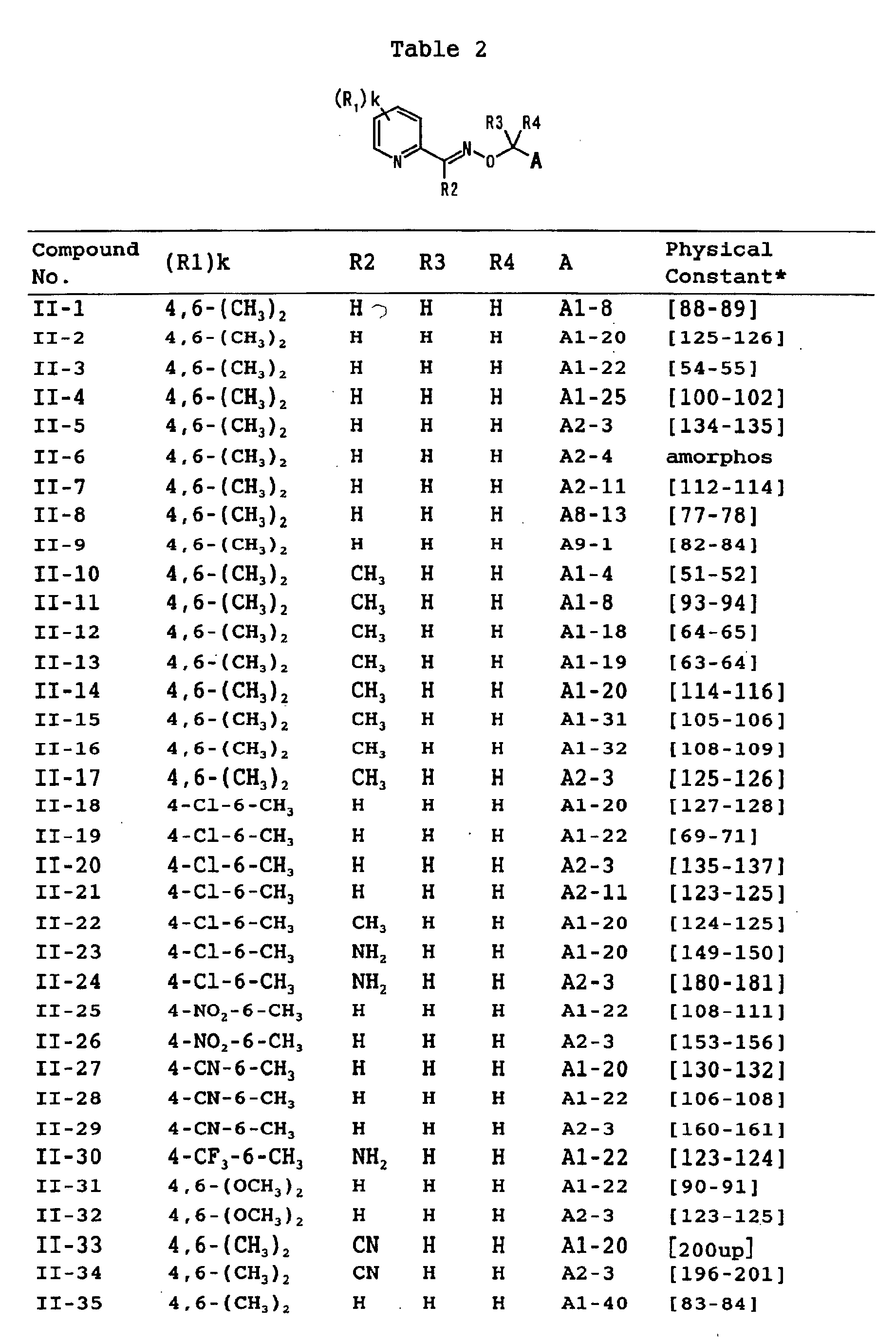

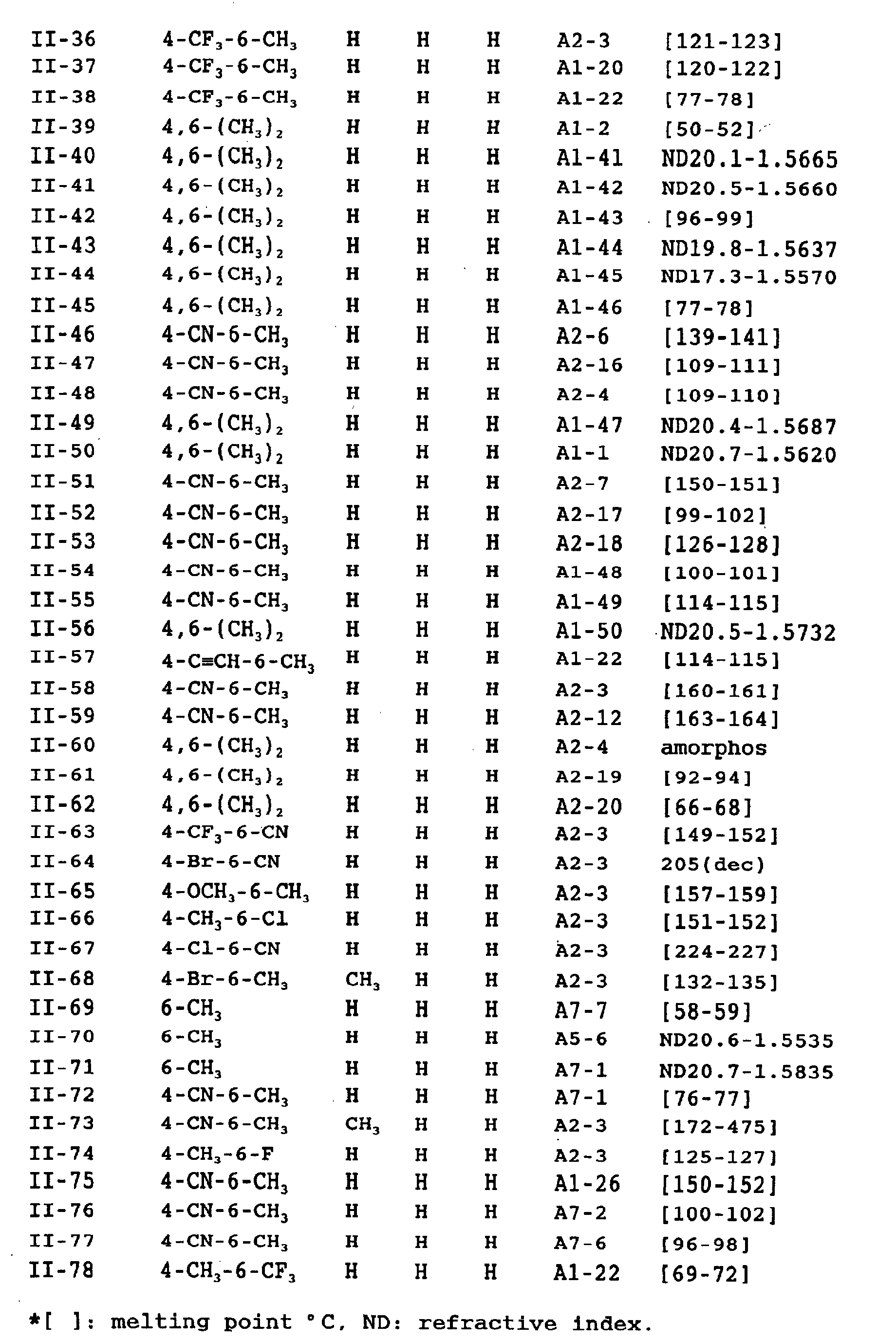

### [Fungicides]

A fungicide of the present invention contains one or more compounds of the present invention as active ingredients.

An obtained compound of the present invention can be used in a pure form without other ingredients added when it is actually applied, or can be used in the form of wettable powder, granule, dust, emulsifiable concentrate, water soluble powder, suspension concentrate, flowable concentrate and other forms which agricultural chemicals can take, when the compound is applied as an agricultural chemical.

Examples of additives and carriers that can be added to agricultural chemical formulations include, for solid formulations, vegetable powders such as soy flour and wheat flour; fine mineral powders such as diatomaceous earth, apatite, gypsum, tulc, bentonite, pyrophyllite and clay; and organic and inorganic compounds such as sodium benzoate, urea and Glauber's salt. In case that the compounds of the present invention are prepared to liquid type formulations, petroleum fractions such as kerosene, xylene and solvent naphtha, cyclohexane, cyclohexanone, dimethylformamide, dimethyl sulfoxide, alcohols, acetone, trichloroethylene, methyl isobutyl ketone, mineral oils, vegetable oils, water and others, can be used as solvents.

To make these formulations uniform and stable, surface active agents may be added, if required. Examples of suitable surface active agents for use include nonionic surface active agents such as polyoxyethylene-added alkylphenyl ethers, polyoxyethylene-added alkyl ethers, polyoxyethylene-added higher fatty acid esters, polyoxyethylene-added sorbitan higher fatty acid esters and polyoxyethylene-added tristyryl phenyl ether; sulfate esters of polyoxyethylene-added alkyl phenyl ethers, alkyl benzene sulfonates, sulfate esters of higher alcohols, alkyl naphthalene sulfonates, polycarboxylates, lignin sulfonates, formaldehyde condensates of alkyl naphthalene sulfonates and copolymers of isobutylene and maleic anhydride.

The content of an active ingredient in an agricultural chemical formulation is 0.01 to 90% by weight, preferably 0.05 to 85% by weight, based on the total weight of the composition (formulation).

A fungicidal composition of the present invention that is formulated according to the method mentioned above is applied to plants, seeds, water surfaces or soil, either as it is or diluted with water or the like. A wettable powder, emulsifiable concentrate or floable is used as a suspension or emulsion by diluting with water to a specified concentration, and a dust and granules are sprayed over plants as they are.

An application dose differs depending on climate conditions, a type of formulation used, when, how and where the fungicide is applied, diseases to be controlled, target crops and other conditions. The dose is usually 1 to 1,000 g, preferably 10 to 100 g, based on the amount of the active ingredient per hectare. When a wettable powder, emulsifiable concentrate, floable, flowable concentrate, liquid formulation or the like is diluted with water for application, the concentration of the active ingredient in the dilution ranges from 1 to 1,000 ppm, preferably from 10 to 250 ppm.

It goes without saying that a compound of the present invention is sufficiently effective on its own as a fungicide, and can also be used by mixing with one or more of various fungicides, insecticides, acaricides or synergists.

Representative examples of fungicides, insecticides, acaricides and plant growth regulators that can be mixed with the compounds of the present invention are shown in the following:

### [Fungicides]

- Copper-based fungicides: Basic copper chloride, Basic copper sulfate, etc.
- Sulfur-based fungicides: Thiuram, Zineb, Maneb, Mancozeb, Ziram, Propineb, Polycarbamate, etc.
- Polyhaloalkylthio-type fungicides: Captan, Folpet, Dichlorfluanid, etc.
- Organochloric fungicides: Chlorothalonil, Futharide, etc.
- Organophosphorous fungicides: IBP, EDDP, Triclofos methyl, Pyrazophos, Fosetyl, etc.
- Benzimidazole fungicides: Thiophanate methyl, Benomyl, Carbandazim, Thiabendazol, etc.
- Dicarboxyimide fungicides: Iprodione, Procymidone, Vinclozolin, Fluorimide, etc.
- Carboxyamide fungicides: Oxycarboxine, Mepronil, Flutolanil, Tecloftalam, Trichlamide, Pencycuron, etc.
- Acylalanine fungicides: Metalaxyl, Oxadixyl, Fralaxyl, etc.
- Methoxyacrylate fungicides: Clethoxime methyl, Azoxystrobine, Methominostrobine, etc.
- Anilinopyrimidine fungicides: Andopurine, Mepaniprim, Pyrimethanil, Diprodinyl, etc.
- SBI fungicides: Triadimefon, Triadimenol, Bitertanol, Microbutanil, Hexaconazole, Propiconazole, Triflumizole, Prochloraz, Beflazoate, Fenarimol, Pyrifenox, Triforine, Flusilazole, Etaconazole, Dicloputrazole, Fluotrimazole, Flutriafen, Penconazole, Diniconazole, Imazalyl, Tridemorph, Fenpropimorph, Buthiobate, Epoxyconazole, Metoconazole, etc.
- Antibiotics: Polyoxins, Blastocidin-S, Kasugamycin, Validamycin, Dihydrostreptomycin sulfate, etc.
- Others: Propamocarb hydrochloride, Quintozene, Hydroxyisoxazole, Metasulfocarb, Anilazine, Isoprothiolane, Probenazole, Quinomethionate, Dithianone, Dinocap, Diclomezine, Ferimzone, Fluazinam, Pyroquilon, Tricyclazole, Oxolinic acid, Dithianone, Iminoctadine acetate, Cymoxanil, Pyrrolenitrine, Matasulfocarb, Diethofencarb, Binapacryl, Lecithin, Sodium bicarbonate, Fenaminosulf, Dodine, Dimetomorph, Fenazine oxide, Carpropamide, Flusulfamide, Fludioxonil, Famoxadone, etc. Insecticides and Acaricides:

- Organophosphorus and carbamate insecticides: Fenthion, Fenitrothion, Diazinon, Chlorpyrifos, ESP, Bamidothion, Fenthoate, Dimethoate, Formothion, Malathon, Trochlorfon, Thiometon, Phosmet, Dichlorvos, Acephate, EPBP, Methyl parathion, Oxadimeton methyl, Ethion, Salithion, Cyanophos, Isoxathione, Pyridafenthion, Phosalone, Methidathion, Sulprofos, Chlorfenvinphos, Tetrachlorvinphos, Dimethylvinphos, Propaphos, Isofenphos, Ethyl thiometon, Profenophos, Pyraclofos, Monocrotophos, Azinphos methyl, Aldicarb, Methomyl, Thiodicarb, Carbofuran, Carbosulfan, Benflacarb, Flathiocarb, Propoxur, BPMC, MTMC, MIPC, Carbaryl, Pyrimicarb, Ethiofencarb, Fenoxycarb, etc.
- Pyrethroid insecticides: Permethrin, Cypermethrin, Deltamethrin, Fenvalerate, Fenpropathrin, Pyrethrin, Allethrin, Tetramethrin, Resmethrin, Dimethrin, Propathrin, Fenothrin, Prothrin, Fluvarinate, Cyfluthrin, Cyhalothrin, Flucythrinate, Ethofenprox, Cycloprothrin, Tralomethrin, Silafluofen, Profenprox, Acrinathrin, etc.
- Benzoyl urea and other insecticides: Diflubenzuron, Chlorfluazuron, Hexaflumuron, Triflumuron, Tetrabenzuron, Fulfenoxuron, Flucycloxuron, Buprofezin, Pyriproxyfen, Methoprene, Benzoepin, Diafenthiuron, Acetamiprid, Imidacloprid, Nitenpyram, Fipronyl, Cartap, Thiocyclam, Bensultap, Nicotine sulfate, Rotenone, Metaldehyde, Machine oil, Microbial insecticides such as BT and insect-pathogenic viruses, etc.
   Nematicides: Fenamiphos, Fosthiazate, etc.

### Acaricides:

Chlorbenzilate, Fenisobromolate, Dicofol, Amitraz, BPPS, Benzomate, Hexythiazox, Fenbutatin oxide, Polynactin, Quinomethionate, CPCBS, Tetradifon, Avermectin, Milbemectin, Clofentezin, Cyhexatin, Pyridaben, Fenpyroximate, Tebufenpyrad, Pyrimidifen, Fenothiocarb, Dienochlor, etc.

### Plant Growth Regulators:

Gibberellins (e.g, Gibberellin A3, Gibberellin A4, Gibberellin A7), IAA, NAA, etc.

### [Fungicides]

Some examples of compositions containing the compounds of the present invention are described. Additives and addition ratios are, however, not limited to these examples and can be changed in a wide range. The term "part" used in the formulation examples stands for °part by weight".

### Example 15: Wettable Powder Formulation

| | |
|---|---|
| A compound of the present invention | 40 parts |
| Clay | 48 parts |
| Sodium dioctylsulfosuccinate | 4 parts |
| Sodium lignin sulfonate | 8 parts |

The above-recited components are mixed uniformly and pulverized to fine particles to thereby obtain a wettable powder formulation for the compound of the present invention with the content of 40% based on the active ingredient.

### Example 16: Emulsifiable Concentrate Formulation

| | |
|---|---|
| A compound of the present invention | 10 parts |
| Solvesso 200 | 53 parts |
| Cyclohexanone | 26 parts |
| Calcium dodecylbenzene sulfonate | 1 part |
| Polyoxyethylene alkylallyl ether | 10 parts |

The above-recited components are mixed and dissolved to thereby obtain an emulsifiable concentrate formulation for the compound of the present invention with the content of 10% based on the active ingredient.

### Example 17: Dust Formulation

| | |
|---|---|
| A compound of the present invention | 10 parts |
| Clay | 90 parts |

The above-recited components are mixed uniformly and pulverized to fine particles to thereby obtain a dust formulation for the compound of the present invention with the content of 10% based on the active ingredient.

### Example 18: Granule Formulation

| | |
|---|---|
| A compound of the present invention | 5 parts |
| Clay | 73 parts |
| Bentonite | 20 parts |
| Sodium dioctylsulfosuccinate | 1 part |
| Potassium phosphate | 1 part |

The above-recited components are mixed, thoroughly grinded, kneaded with water added, then granulated, and further dried to thereby obtain a granule formulation for the compound of the present invention with the content of 5% based on the active ingredient.

### Example 19: Flowable Concentrate Formulation

| | |
|---|---|
| A compound of the present invention | 10 parts |
| Polyoxyethylene alkylally ether | 4 parts |
| Sodium polycarboxylate | 2 parts |
| Glycerin | 10 parts |
| Xanthane gum | 0.2 parts |
| Water | 73.8 parts |

The above-recited components are mixed, and wet pulverized until the granule size becomes smaller than 3µm to thereby obtain a flowable concentrate formulation for the compound of the present invention with the content of 10% based on the active ingredient.

### Example 20: Water Dispersible Granule Formulation

| | |
|---|---|
| A compound of the present invention | 40 parts |
| Clay | 36 parts |
| Potassium chloride | 10 parts |
| Sodium alkylbenzene sulfonate | 1 part |
| Sodium lignin sulfonate | 8 parts |
| Formaldehyde polycondensate of sodium alkylbenzene | |
| sulfonate | 5 parts |

The above-recited components are mixed uniformly and pulverized to fine particles. An appropriate amount of water is added to the mixture to knead to be clay like. The obtained clay-like product is granulated and dried to thereby obtain a water-dispersible granule formulation for the compound of the present invention with the content of 40% based on the active ingredient.

### Applicability in Industry:

The following test examples show the efficacy of the compounds of the present invention as active ingredients of various agents for controlling plant diseases. The control efficacy was determined by visual observation of the infested states of test plants, that is, degrees of legions and growth of colonies on leaves and stalks, when they were examined, in comparison with those of control plants.

### Test Example 1: Test on Apple Scab Control (Test on Prevention)

Apple seedlings (variety: Kokko, 3 to 4 leaf stages) grown in clay pots were sprayed with a solution of an emulsifiable concentrate prepared for a compound of the present invention at a concentration of 200 ppm based on the active ingredient. The seedlings were dried at room temperature, and inoculated with conidia of apple scab fungus *(Venturia inaequalis).* The treated seedlings were placed in a room kept at 20°C with high humidity and repeated lighting of 12-hour intervals, and incubated for 2 weeks. The infestation degrees by the fungus on the leaves were examined in comparison with those of the control healthy leaves to determine the control efficacy. As a result, the compounds listed below showed excellent efficacy of 75% or more. The compound numbers correspond to those shown in Table 2.

### Compound Numbers: II-3, II-12, II-14, II-15, II-43, II-44.

### Test Example 2: Test on Kidney Bean Gray Mold Control

Flowers of kidney bean (variety: Nagauzura) grown in a flat vessel for culturing seedlings were cut, and dipped into a solution of an emulsifiable concentrate prepared for a compound of the present invention , diluted to a concentration of 50 ppm based on the active ingredient. After the dipping, the flowers were dried at room temperature. Then a spore solution of kidney bean gray mold fungus *(Botrytis cinerea)* was sprayed onto the flowers. The treated flowers were placed on leaves detached from healthy kidney bean plants, placed in a temperature-controlled room at 20°C with high humidity and repeated lighting of 12-hour intervals, and incubated for 7 days. Diameters of the legions on the leaves were measured and compared with those of the control healthy leaves to determine the control efficacy. As a result, the compounds listed below showed excellent efficacy of 75% or more. The compound numbers correspond to those shown in Table 2.

Compound Numbers: II-2, II-3, II-5, II-7, II-10, II-11, II-12, II-13, II-14, II-17, II-18, II-19, II-20, II-21, II-27, II-28, II-29, II-36, II-37, II-38, II-54, II-58, II-59, II-61, II-62, II-63, II-66, II-78

## Claims

1. An oxime ether compound represented by Formula [I] 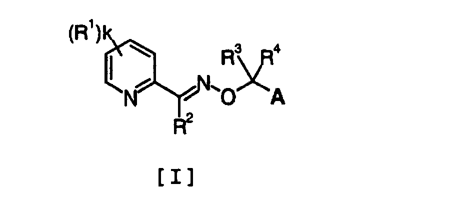 [wherein, R¹ is halogen, hydroxyl, C₁₋₆ alkyl, C₁₋₆ haloalkyl, C₁₋₆ alkoxy, C₁₋₆ alkoxy C₁₋₆ alkyl, cyano, nitro, C₂₋₆ alkenyl, C₂₋₆ haloalkenyl, C₂₋₆ alkynyl, amino, mono-C₁₋₆ alkylamino, di-C₁₋₆ alkylamino, C₃₋₆ cycloalkyl, carboxyl, C₁₋₆ alkoxycarbonyl, C₁₋₆ alkylthio, C₁₋₆ alkylsulfinyl or C₁₋₆ alkylsulfonyl;
R² is hydrogen, C₁₋₆ alkyl, C₃₋₆ cycloalkyl, C₁₋₆ haloalkyl, amino or cyano;
R³ and R⁴ are, the same or different, hydrogen or C₁₋₆ alkyl;
k is an integer of 1 to 4; when k is 2 or larger, R¹ may be the same or different; and
A is a heterocyclic group represented by the following Formulae A-1 to A-12 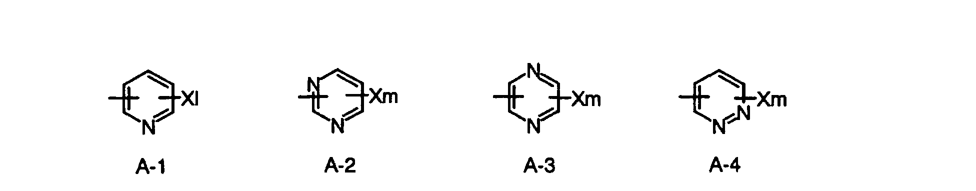 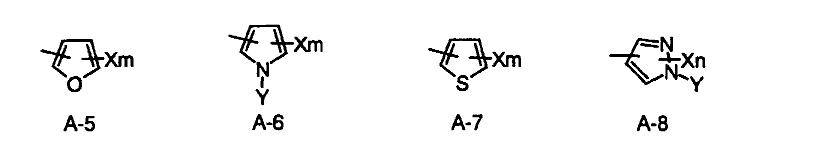 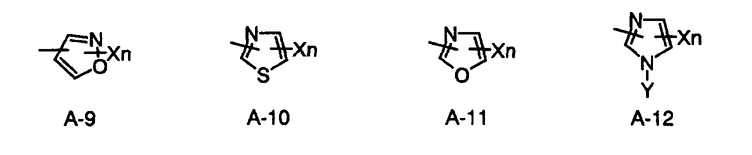 (wherein, X is halogen, C₁₋₆ alkyl, C₁₋₆ haloalkyl, hydroxyl, C₁₋₆ alkoxy, cyano, amino, mono-C₁₋₆ alkylamino, di-C₁₋₆ alkylamino, C₃₋₆ cycloalkyl, C₁₋₆ alkylcarbonyloxy or C₁₋₆ alkylthio; 1 is 0 or an interger of 1 to 4; m is 0 or an integer of 1 to 3; n is 0, 1 or 2; when 1, m and n are 2 or more, X may be the same or different; but when A is A-7, m is not 0; and Y is hydrogen or C₁₋₆ alkyl)] or a salt thereof.

2. An agricultural or horticultural fungicide containing one or more of the oxime ether compounds represented by Formula [I] 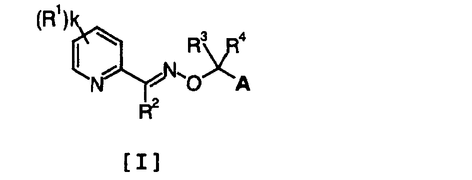 (wherein, R¹, R², R³, R⁴, A and k are as defined above) or salts thereof as active ingredients.
